# FASCICULE DE BREVET EUROPEEN

(11) **EP 1 546 366 B1**
(45) Date de publication et mention de la délivrance du brevet: **17.09.2008**
(21) Numéro de dépôt: 03775457.9
(22) Date de dépôt: 01.10.2003
(51) Int. Cl.: C12Q 1/04, C12Q 1/06

(54) **PROCEDE DE DETECTION ET DE COMPTAGE DE MICROORGANISMES DANS UN ECHANTILLON**
VERFAHREN ZUR DETEKTION UND ZÄHLUNG VON MIKROORGANISMEN IN EINER PROBE
METHOD FOR DETECTING AND COUNTING MICRO-ORGANISMS IN A SAMPLE

(30) Priorité: 01.10.2002 FR 0212119
(43) Date de publication de la demande: 29.06.2005
(73) Titulaire: Metis Biotechnologies, 87069 Limoges cedex (FR)
(72) Inventeur: VEDRINE, Bruno, F-63230 Pontgibaud (FR); LACHAISE, Aline, F-87370 Rilhac-Rancon (FR); CARRE, Vincent, F-87370 Jabreilles-Les-Bordes (FR)
(74) Mandataire: Warcoin, Jacques
(86) Numéro de dépôt international: PCT/FR2003/002873
(87) Numéro de publication internationale: WO 2004/031403

(56) Documents cités:
- EP-A- 0 877 092
- WO-A-89/04372
- WO-A-95/31481
- WO-A-99/02650
- GB-A- 1 554 193
- US-A- 5 534 415
- US-A- 6 120 987
- BAILEY J S ET AL: "UNIVERSAL PREENRICHMENT BROTH FOR THE SIMULTANEOUS DETECTION OF SALMONELLA AND LISTERIA IN FOODS" JOURNAL OF FOOD PROTECTION, DES MOINES, IO, US, vol. 55, no. 4, avril 1992 (1992-04), pages 256-259, XP002925366 ISSN: 0362-028X
- EDBERG S C ET AL: "RAPID SPOT TEST FOR THE DETERMINATION OF ESCULIN HYDROLYSIS" JOURNAL OF CLINICAL MICROBIOLOGY, vol. 4, no. 2, 1976, pages 180-184, XP009026425 ISSN: 0095-1137

## Description

La présente invention a pour objet un procédé de détection et de comptage de microorganismes dans un échantillon ainsi qu'un milieu d'enrichissement sélectif du microorganisme recherché.

La présente invention s'applique à la détection de microorganismes tels que *Listeria monocytogenes, Salmonella, Staphylococcus aureus, Escherichia coli, Mycobacterium avium paratuberculosis* et *Legionella pneumophila.* Cette détection est effectuée préférentiellement par cytométrie en flux à partir d'échantillons d'une quelconque nature tels que les échantillons alimentaires, solides, liquides, végétaux, animaux... ainsi que des échantillons de tissus animaux et, en particulier, humains.

Les contaminations microbiennes dans l'industrie agroalimentaire sont une préoccupation majeure, car elles sont à l'origine d'importantes dégradations et d'une immobilisation des marchandises dans l'attente d'une analyse libératoire. Les critères attendus des analyses microbiologiques sont de deux ordres. D'une part, il faut pouvoir contrôler le plus précisément possible l'état de la contamination, ce qui nécessite d'avoir des méthodes d'analyses très sensibles et discriminantes. D'autre part, ces méthodes doivent être rapides pour permettre une libération anticipée des lots de production, et doivent rendre possible une analyse microbiologique prédictive.

Le critère de rapidité est déterminant si l'on veut éviter de commercialiser un produit à risque. Les analyses classiques nécessitent toujours un temps d'incubation très long par rapport aux exigences d'une production alimentaire. De plus, elles ne prennent pas en compte les bactéries qui ont perdu leur aptitude à se multiplier, alors que celles-ci conservent des activités métaboliques susceptibles de se réveiller à la faveur d'un évènement particulier tel que la remontée en température suite à une rupture de la chaîne du froid ou le passage dans l'organisme du consommateur.

Par ailleurs, dans l'environnement, la rapidité et la sensibilité des méthodes d'analyses microbiologiques appliquées aux eaux techniques ou de consommation sont déterminantes, ces eaux pouvant contaminer directement les personnes (eaux de consommation) ou indirectement par pollution de l'environnement (légionnelles dans l'air climatisé, contamination des surfaces en contact avec les aliments).

Parmi les techniques microbiologiques classiques pour la détection et le comptage des microorganismes, la culture en boîte de Pétri est très largement utilisée.

La méthode de comptage sur boîte de Pétri, est la méthode conventionnelle d'estimation des populations microbiennes. La croissance et la division sont les fondements de cette méthode d'analyse microbiologique pour rechercher les bactéries viables. Dans ce cas, la cultivabilité est associée à la viabilité. La cultivabilité, en fait, est définie par la capacité d'une seule cellule à donner une population observable ou visible à la surface d'une boîte de culture.

Cette technique est utilisée depuis des décennies et a notamment montré son efficacité dans la protection de la santé des personnes à un coût relativement faible. Ces dernières années, de nombreuses innovations ont porté sur la conception des milieux de culture (milieux chromogènes), l'automatisation de la préparation des milieux de culture, la dilution de l'échantillon broyé dans le milieu, l'étalement et le comptage des colonies sur boîte de Pétri (Garcia-Armesto *et al.,* 1993). Cependant, elle est défaillante sur deux aspects. Tout d'abord, elle est peu sensible car, dans son principe, elle nécessite de faire pousser les bactéries sur des milieux nutritifs dans une boîte de Pétri, lesdits milieux nutritifs étant d'une efficacité variable pour la pousse sélective des germes, en fonction du produit agroalimentaire testé. Ensuite, la culture en boîte de Pétri nécessite que les germes recherchés soient dans des conditions physiologiques optimales pour pouvoir pousser dans un milieu nutritif sélectif. Or, il n'est pas rare que des bactéries présentes initialement dans un échantillon ne soient plus détectables par les techniques classiques de microbiologie, à savoir par la culture dans des milieux standards, et ceci peut s'expliquer de deux façons : soit les bactéries sont mortes à la suite d'un stress trop important (choc thermique, osmotique, manque de nutriments...), soit elles ont perdu leur capacité à proliférer dans les milieux de culture standard ; on dit alors que les bactéries sont viables mais non cultivables (Barer *et al.,* 1999). Il est donc important de pouvoir détecter ces bactéries dans un échantillon.

Récemment, il a été montré que quelques espèces cultivables de bactéries, lorsqu'elles sont soumises à un manque de nutriments ou à un autre stress physique (thermique, hydrique,...), peuvent entrer dans un état de survie cellulaire dans lequel elles ne sont pas détectables par des tests de cultivabilité (par exemple par culture sur boîte de Pétri).

C'est pourquoi, les techniques d'amplification de l'ADN, qui apportent une plus grande spécificité vis-à-vis des microorganismes recherchés et qui sont automatisables, ont été développées. Ces techniques consistent en la détection de séquences nucléotidiques spécifiques d'un microorganisme (après amplification génique). Cette approche ne permet cependant pas encore de compter les microorganismes dans un échantillon.

En terme de spécificité, les sondes génétiques (ADN, ARN, PNA, molecular beacons...) sont une alternative à l'utilisation d'anticorps fluorescents. Ces sondes doivent être marquées à l'aide d'un fluorochrome et s'hybrider sur les acides nucléiques des cellules étudiées sans que celles-ci n'aient été lysées au préalable : il s'agit alors d'hybridation *in situ.* Dans ce cas, les sondes utilisées ciblent l'ARN 16S ou 23S, ce qui permet à la fois d'assurer la spécificité de la détection et d'augmenter le signal lié au nombre de copies de cet ARN dans la cellule (Moter *et al.,* 2000).

D'autres techniques, telles que les méthodes immunologiques (dont les méthodes Elisa) consistent en la détection à l'aide d'anticorps des antigènes microbiens, par luminescence ou fluorescence. Si ces méthodes sont facilement automatisables et sensibles, elles donnent une information restreinte puisqu'elles ne permettent pas de compter les microorganismes présents. De plus, ces méthodes sont parfois critiquées sur leur spécificité, les anticorps utilisés pouvant présenter des spectres de reconnaissance d'antigènes microbiens trop restreints (Tortorello *et al.,* 1994).

Quelles que soient les méthodes employées, elles nécessitent toutes une phase d'enrichissement des bactéries recherchées pour obtenir une sensibilité et une spécificité acceptables.

Les analyses cytométriques, dont la cytométrie en flux n'est qu'un exemple, présentent l'avantage de permettre une détection directe des germes qui sont, au préalable, marqués par une molécule fluorescente. De plus, ce marquage peut rendre compte de la viabilité de la bactérie. La détection par un cytomètre en flux est directe et quantitative et ne prend que quelques minutes.

La cytométrie en flux, par son succès d'adaptation à la biologie médicale, est très bien positionnée pour une adaptation à l'analyse microbiologique à haut débit (Alvarez-Barrientos *et al.,* 2000 ; Davey *et al.,* 1996). Une des caractéristiques techniques de la cytométrie en flux est qu'elle peut permettre, en moins de 24 h/ 48 h, des analyses de populations définies par des caractéristiques particulières de croissance (coliformes fécaux à 44°C, flores psychrophiles), qui après enrichissement dans un bouillon sélectif, peuvent être détectées directement par cytométrie en flux, au lieu d'attendre qu'elles poussent sur un milieu spécifique.

Ces dernières années, de nombreux cytomètres commerciaux inutilisables pour la détection des microorganismes ont été dévelopés pour l'analyse des cellules eucaryotes. Mais l'évolution des photomultiplicateurs, l'introduction des lasers et l'amélioration des systèmes optiques (focalisation, cellules d'analyses en quartz) permettent aujourd'hui à la plupart des cytomètres de quantifier des bactéries et des virus (Salzman *et al.,* 1982, Steen *et al.,* 1986). Le progrès le plus significatif a consisté en l'adoption d'une mesure de la taille des cellules procaryotes ou eucaryotes analysées, par une photodiode tout d'abord, puis par un photomultiplicateur. Ainsi, il est désormais possible de sélectionner pour l'analyse de la fluorescence émise une population cellulaire de taille définie (les microorganismes par exemple). La fenêtre d'analyse ainsi créée permet donc de concentrer la puissance d'analyse du cytomètre sur des évènements prédéfinis, permettant d'obtenir une bonne différenciation entre le signal de fluorescence émis par le microorganisme et le bruit de fond de la matrice.

Les marqueurs fluorescents utilisés marquent des populations microbiennes dans leur globalité et sont d'un spectre d'action large : des intercalants de l'ADN, des marqueurs de potentiels membranaires ou de l'activité respiratoire des bactéries (Lopez-Amoros *et al.,* 1995 ; Grepori *et al.,* 2001). Aux signaux de fluorescence est associée la mesure de la taille et de la granulosité du contenu intracellulaire. L'utilisation de molécules fluorescentes pour détecter des microorganismes viables par cytométrie en flux est à présent bien connue (Davey *et al.,* 1996 ; Nebe-von Caron *et al.,* 1995). Le principe est de détecter une activité enzymatique présente chez tous les microorganismes. Ce sont dans tous les cas des activités à spectre très large et essentielles à la survie cellulaire. De multiples exemples sont décrits dans la littérature, les plus pertinents sont les substrats permettant de mettre en évidence l'ensemble des activités estérasiques comme la fluorescéine-di-acétate (FDA) ou son homologue la carboxy-fluorescéine-di-acétate (CFDA) (Breeuwer *et al.,* 1995).

Drocourt et al. (FR 2 764 305) décrivent un procédé de marquage de tous les microorganismes viables (marquage FDA et CFDA) dans un échantillon complexe, basé sur la détection des activités estérasiques et surtout la suppression à l'aide d'un contre-colorant de l'autofluorescence de l'échantillon.

Breeuwer et al. (WO05/00660) décrivent un procédé de mesure de la viabilité des microorganismes dans un échantillon, en utilisant des marqueurs fluorescents. Le procédé consiste à mesurer le temps requis pour qu'il se produise une perte de la moitié de la fluorescence émise par ces microorganismes.

Inoue et al. (EP 1 203 825) utilisent la polyméthine pour révéler une activité réductrice des ions nitrites dans les microorganismes. C'est une activité cellulaire *a priori* détectable chez toutes les bactéries.

Rocco et al. (WO00/34509) utilisent des antibiotiques sur lesquels sont greffés des molécules fluorescentes pour révéler les microorganismes.

Fazzi et al. (WO00/067065) ont mis au point une méthode qui permet de différencier les bactéries selon leur coloration de Gram, en utilisant un mélange d'acridine orange et de fluorescéine. La détection des populations microbiennes marquées se fait alors par cytométrie en flux.

Jespersen et al. (WO02/00036) ont également développé une méthode de coloration de Gram basée sur l'utilisation de molécules reconnaissant des différences structurales entre les bactéries à Gram positif (structures glycanniques à la surface des cellules) ou négatif (présence du lipopolysaccharide). Cependant, cette méthode ne permet pas la mesure de la viabilité des bactéries.

Pyle et al. (WO95/31481) utilisent le CTC comme molécule révélatrice de l'activité respiratoire des microorganismes. Cette détection est couplée à un enrichissement immuno-magnétique et/ou à une détection immunologique à l'aide d'anticorps marqués à la fluorescéine.

Cependant, les marqueurs fluorescents de viabilité cités ci-dessus ont un spectre beaucoup trop large pour apporter la spécificité nécessaire à la détection de microorganismes spécifiques.

L'association des technologies de biologie moléculaire (utilisation de sondes nucléotidiques marquées en fluorescence) à la cytométrie en flux est une piste qui a été engagée assez récemment (Amann *et al.,* 1995 ; Wallner *et al.,* 1995).

Cependant, si elle fonctionne bien pour des microorganismes à Gram négatif, des recherches sont encore nécessaires pour marquer spécifiquement les bactéries à Gram positif. Vlieger *et al.* (1992) décrivent l'utilisation de la cytométrie en flux pour détecter les acides nucléiques amplifiés par PCR par hybridation desdits fragments à des billes.

La cytométrie en flux a notamment été utilisée pour dénombrer les bactéries totales ou les levures dans du lait et des préparations de viandes, du lait fermenté, des végétaux surgelés ou du lait cru. Les marqueurs utilisés sont des marqueurs de viabilité (Breeewer *et al.,* 1995). A ce premier critère de sélection sont ajoutés les critères morphologiques de taille et de structure, qui sont analysables par cytométrie en flux.

La détection plus spécifique de microorganismes (genres et espèces) dans les aliments concerne en particulier les flores pathogènes telles que *Salmonella, Listeria monocytogenes, Escherichia coli* 0157:H7, *Staphylococcus aureus, Brucella abortus* (McClelland *et al.,* 1994 ; Iannelli *et al.,* 1998 ; Pinder *et al.,* 1994 ; Skjerve *et al.,* 1990, 1991 ; Tomayasu *et al.,* 1998 ; Tortorello *et al.,* 1998). Les marqueurs fluorescents utilisés pour une identification des genres bactériens demeurent les anticorps, pour lesquels les protocoles sont nombreux et variés, mais peu sensibles s'ils sont utilisés en direct sur les produits. En effet, la numération d'un petit nombre d'évènements cellulaires dans un aliment est difficile, à cause de la présence d'un bruit de fond important dû à la matrice et à la flore endogène, et au choix du marqueur fluorescent pour obtenir une réelle information sur la viabilité des cellules. Ainsi, il est recommandé de multiplier les marqueurs fluorescents (anticorps, sondes stoechiométriques, intercalants de l'ADN, substrats enzymatiques...) pour discriminer entre une bactérie et des particules fluorescentes entre des espèces de bactéries différentes.

Pour améliorer la détection du genre et de l'espèce bactérienne, la séparation immunomagnétique a été utilisée avec succès pour séparer des microorganismes pathogènes d'une matrice alimentaire complexe. Les sociétés Dynal (Oslo, Norvège) et Vicam (Watertown, USA) commercialisent des billes magnétiques sur lesquelles sont greffés des anticorps qui reconnaissent des bactéries pathogènes. Cependant, des applications conventionnelles nécessitent toujours un étalement sur boîte de Pétri après incubation dans un milieu d'enrichissement sélectif, et parfois des tests de confirmation. Cela réside essentiellement dans le manque de sélectivité des anticorps utilisés ou des interactions non spécifiques de la flore naturelle avec le support de la bille magnétique (Tomayasu *et al.,* 1998). Par exemple, il est reconnu que des souches de *Vibrio parahaemolyticus* peuvent s'adsorber à la surface des billes magnétiques de manière non-spécifique et que cette capacité d'adsorption est dépendante de l'identité des souches de *Vibrio.* Des améliorations de structure de ces billes paramagnétiques ont consisté à encapsuler la bille dans une structure chimique moins réactive avec le milieu (encapsulated nanobeads, Estapor).

La séparation immuno-magnétique a montré son efficacité à récupérer des cellules eucaryotes à partir de liquides, mais aussi à isoler des microorganismes à partir d'échantillons aussi variés que des aliments, du lait, des fèces (Grant *et al.,* 1998 ; Skjerve *et al.,* 1990, 1991, Pinder *et al.,* 1994 ; Seo *et al.,* 1998) Des billes paramagnétiques présentant à leur surface des anticorps dirigés contre des antigènes de la surface membranaire sont commercialisées. Elles permettent de concentrer des bactéries à partir d'un échantillon complexe, améliorant la sensibilité des techniques utilisées ultérieurement comme la PCR, l'hybridation de sondes nucléiques, l'immunofluorescence et la cytométrie en flux. Cette immuno-concentration réalisée par l'isolement des bactéries fixées aux billes sur la paroi d'un tube permet dans un même temps d'éliminer les agents inhibiteurs présents dans l'échantillon. Elle constitue de plus une phase sélective.

L'utilisation de l'immunofluorescence couplée à la cytométrie en flux répond bien à ce problème de spécificité. Les propriétés immunologiques des bactéries pathogènes des aliments sont connues depuis longtemps et largement utilisées dans des tests de détection rapides. Ces anticorps spécifiques marqués au FITC ont été utilisés pour marquer des Salmonelles ajoutées dans des échantillons de lait et d'oeufs ou dans des eaux de rinçage de carcasses de poulets contaminées (McClelland *et al.,* 1994). Le couplage immunofluorescence et cytométrie en flux a également été appliqué pour détecter *Escherichia coli* 0157:H7 dans des échantillons de viande artificiellement contaminés après destruction de la flore naturelle (Seo *et al.* 1998).

Cependant, dans tous les cas, un pré-traitement de l'échantillon est nécessaire avant l'analyse (traitement chimique ou immuno-séparation) et le seuil de détection ne descend pas en dessous de 10 000 cellules/g de produit, ce qui est trop élevé par rapport aux exigences relatives à la détection des bactéries pathogènes dans les aliments.

Forrest *et al.* 1979 (US 4,141,687) et Ithakissios *et al.* 1978 (US 4,115,534) décrivent la synthèse de particules magnétiques permettant par couplage de molécules de reconnaissance d'extraire par l'application d'un champ magnétique des molécules, des cellules eucaryotes ou procaryote d'un mélange complexe.

Coulter Electronics (UK 1,561,042) décrit l'utilisation de la cytométrie en flux pour isoler et analyser des particules et des cellules liées à ces particules.

Pinder et al. (WO98/20214) ont développé des billes magnétiques de taille inférieure à 1 micron (100 nm étant la taille préférée), qui sont fluorescentes. L'intérêt d'utiliser de telles billes pour extraire et marquer des micro-organismes réside dans leur petitesse. Il n'est pas nécessaire de décrocher lesdites billes des micro-organismes pour pouvoir les détecter par cytométrie en flux.

Pyle et al. (WO 95/31481) utilisent des billes magnétiques pour concentrer des bactéries à partir d'un échantillon complexe. Ces bactéries sont alors marquées en vert par un anticorps fluorescent (fluorescéine par exemple) et l'activité respiratoire des cellules est détectée par le CTC (Cyano Tetrazolium Chloride) qui induit une fluorescence rouge chez les cellules, la détection se faisant par microscopie de fluorescence.

Senyei et al. (EP/0016552) ont greffé sur des billes magnétiques la protéine A (qui peut se lier à de nombreux anticorps) pour ensuite récupérer des cellules ayant à leur surface un anticorps lié.

Vesey et al. (WO96/31777) analysent par cytométrie en flux les complexes formés par des billes de latex fixées à des micro-organismes par l'intermédiaire d'anticorps fluorescents. Ils décrivent l'utilisation d'un anticorps fluorescent pour marquer les micro-organismes qui ont été extraits de l'échantillon par voie immuno-magnétique.

Fortin et al. (WO99/47933) utilisent des billes de latex de taille comprise entre 0,08 et 0,5 micron sur lesquelles sont greffés des anticorps reconnaissant des cellules. Il se forme ainsi des complexes observables par cytométrie en flux, selon les caractéristiques de taille et de structure.

La détection de microorganismes dans un échantillon peut également être réalisée par la détection d'activités enzymatiques spécifiques, par l'utilisation de substrats chromogènes ou fluorescents. La revue de Manafi (2000) est une mise à jour assez complète des stratégies utilisées pour identifier les micro-organismes sur la base de la présence ou non d'activités enzymatiques particulières dans le microorganisme. Les principales clés dichotomiques sont la mise en évidence d'activités estérasiques particulières (qui n'hydrolysent que des acides gras dont la longueur de la chaîne aliphatique est définie), glycosidiques (hydrolyses de différents sucres), phosphatases et sulfatases. Les substrats utilisés comportent une partie cible, qui est reconnue par l'enzyme recherchée, et une partie marqueur. Cette partie marqueur lorsqu'elle est liée à la cible n'émet de signal visible (chromogène ou fluorescent) qu'après hydrolyse de la liaison marqueur-cible. Les modes de révélation les plus répandus, de ces activités métaboliques sont l'addition de ces substrats dans des milieux de culture. Les substrats, lorsqu'ils sont assimilés par les micro-organismes à l'intérieur d'une colonie, sont responsables d'une coloration de celle-ci. La combinaison des milieux sélectifs et des substrats (un ou plusieurs par milieu) permet d'obtenir une identification précise du micro-organisme recherché, par l'observation de la forme et de la couleur de la bactérie.

Ainsi, pour qu'il y ait coloration d'une colonie sur un milieu chromogène, il faut que le substrat soit expulsé à l'extérieur du microorganisme, à l'intérieur de la colonie.

Schabert et al. (WO99/48899 décrivent une voie de synthèse pour un substrat fluorescent (sous excitation UV) permettant de révéler l'activité phosphatylinositol spécifique de la phospholipase C. Ce substrat (comportant la 4-méthylumbelliferone comme molécule fluorescente) est utilisé dans la conception de deux milieux de culture permettant d'identifier *Listeria monocytogenes* et *Bacillus cereus.* Ce substrat n'est cependant pas assez spécifique d'un genre bactérien pour être utilisé dans le cadre de la présente invention.

Conrad et al. (WO00/03034) ont mis au point des substrats fluorescents comportant l'esculine comme molécule fluorescente. Les méthodes de détection et l'utilisation précise de ces substrats pour identifier les microorganismes ne sont pas décrites.

Berg et al. (US 5,292,644) décrivent l'utilisation de la 4-méthylumbelliferone-β-D-galactosidase, qui est excitable par les UV (λexc= 365 nm), pour détecter chez les bactéries l'activité galactosidase, que ces microorganismes soient cultivés en milieu liquide ou solide. La détection se fait par excitation de la méthylumbelliferone par les U.V., lorsque celle-ci est relarguée dans le milieu extérieur.

Nelis et al. (WO 98/13515) décrivent l'utilisation de substrats chromogènes ou fluorescents consécutivement à une excitation par les U.V., qui permettent de mettre en évidence les activités bêta-D-galactosidase ou bêta-D-glucuronidase chez les micro-organismes. La technique décrite se limite à la détection desdits microorganismes, marqués sur des milieux de culture solides ou retenus sur un filtre.

Rambach Alain (WO 00/53799) utilise les substrats glucose et phosphate greffés à des molécules chromogènes pour détecter dans un milieu spécifique contenant de la déferoxamine, *Staphylococcus aureus.* La détection repose sur l'observation de composés chromogènes dans la colonie bactérienne, des composés qui sont expulsés par les bactéries à l'intérieur de la colonie. L'identification de *Staphylococcus aureus* est basée sur l'observation de composés chromogènes résultants de l'action des enzymes phosphatase et glucosidase.

Cooke et al. (W0 00/41409) marquent spécifiquement les Salmonelles à l'aide d'un substrat chromogène spécifique des activités octanoate-estérases (ou caprylate-estérase) dudit micro-organisme. L'utilisation de l'activité caprylate-estérase comme moyen d'identification de Salmonella est décrite depuis de nombreuses années (Humbert et al. 1989) et montre peu d'efficacité lorsqu'elle couplée avec des milieux de culture solides. Dans la demande de brevet de Cooke *et al.,* un substrat permettant de détecter la β-D-galactosidase est ajouté au milieu afin d'obtenir une plus grande spécificité.

Berg J. (WO89/04372) décrit une méthode de détection de microorganismes comprenant une étape de concentration de l'échantillon par filtration, suivie d'une étape dans laquelle le microorganisme est à la fois enrichi, conditionné puis marqué par fluorescence à l'aide d'un substrat spécifique de l'activité enzymatique induite.

La présente invention se propose de remédier aux inconvénients de l'art antérieur en proposant un procédé de détection et de numération ou comptage de microorganismes dans un échantillon solide ou liquide dans des conditions de temps et de spécificités nettement améliorées.

Dans le cadre de la présente invention, la recherche de microorganismes peut être réalisée à partir d'échantillons d'origine agroalimentaire, tels que des produits carnés, des ovo produits, des eaux, des produits laitiers y compris le lait, des jus de fruits, des produits végétaux et de leurs dérivés, ainsi qu'à partir d'échantillons d'origine animale y compris humaine, tels que tout type de tissu, des fèces et du sang.

Le procédé de l'invention comprend notamment trois étapes sélectives et spécifiques du microorganisme recherché dans un échantillon, à savoir: l'enrichissement sélectif du microorganisme en question, l'activation dudit microorganisme dans un milieu conditionnant et le marquage fluorescent dudit microorganisme.

En fait, le procédé de détection et de numération ou de comptage de microorganismes dans un échantillon conforme à l'invention, comprend 5 étapes dont certaines peuvent être concomitantes et dont l'ordre peut varier en fonction du microorganisme recherché.

Le procédé de détection conforme à l'invention comprend les étapes de :

L'un des objectifs de la présente invention est de palier le manque de sensibilité et de spécificité des méthodes de détection de microorganismes de l'art antérieur et le procédé conforme à l'invention permet d'obtenir des seuils de détection très faibles, de l'ordre de 10 à 100 microorganismes/g d'échantillon, c'est-à-dire des seuils 100 à 1 000 fois plus faibles que ceux de l'art antérieur.

Un autre objectif de l'invention est de permettre une détection des microorganismes en moins de 24 heures, ce qui est notamment obtenu par une étape d'enrichissement et de conditionnement du microorganisme recherché en moins de 18 heures, un marquage fluorescent en moins de 3 heures, et une détection par cytométrie en flux en quelques minutes.

La première étape du procédé conforme à l'invention est une étape d'enrichissement sélectif du microorganisme recherché dans l'échantillon, cette étape ayant un double but. Elle permet non seulement la multiplication dudit microorganisme mais également permet la revivification dudit microorganisme dans le cas où celui-ci est par exemple stressé. Cette étape d'enrichissement est réalisée dans une composition comprenant des nutriments adaptés à la culture du microorganisme recherché sur une durée de 8 à 15 h. Cette composition comprend également des agents chargés de détruire les espèces réactives à l'oxygène et les agents anti-oxydants qui pourraient être présents dans l'échantillon.

Cette composition est également destinée à rendre ou maintenir une certaine viabilité au microorganisme dont le critère de cultivabilité est
a) enrichissement sélectif du microorganisme recherché dans l'échantillon,
b) induction ou activation d'au moins une activité enzymatique dudit microorganisme,
c) concentration immunomagnétique du microorganisme conditionné,
d) marquage fluorescent du microorganisme concentré obtenu après concentration immunomagnétique et réalisé par l'ajout au milieu contenant ledit microorganisme d'au moins un substrat contenant une partie spécifique de l'activité enzymatique à révéler et une partie marqueur, la transformation du substrat ayant lieu a l'intérieur dudit microorganisme et le produit fluorescent étant retenu dans ledit microorganisme, et
e) détection et analyse de la fluorescence permettant la numération ou le comptage des microorganismes recherchés par une technique choisie dans le groupe comprenant la cytométrie en flux, la cytométrie sur filtre et la microscopie de fluorescence. momentanément perdu pour diverses raisons, suite à un stress quelconque tel qu'un traitement thermique par exemple.

L'intérêt d'une telle démarche est de pouvoir ensuite détecter et compter les microorganismes en question afin de les prendre en compte dans l'analyse de l'échantillon car de tels microorganismes sont susceptibles de se « réveiller » ultérieurement à la faveur d'un évènement particulier, tel qu'un changement de température, et devient alors un danger pour la santé du consommateur ou peut donner lieu à une sous-estimation du niveau de contamination ou d'infection dudit échantillon.

Selon un mode de réalisation préféré de l'invention, la composition mise en oeuvre dans l'étape d'enrichissement sélectif du microorganisme recherché dans l'échantillon comprend :
- du pyruvate de sodium à une concentration comprise entre 1 à 20 g/L, de préférence entre 1 à 10 g/L et plus préférentiellement 4 à 6 g/L,
- du thiosulfate de sodium à une concentration comprise entre 0,5 et 5 g/L, de préférence entre 0,5 et 3 g/L et plus préférentiellement encore environ 2 g/L,
- de la catalase à une concentration comprise entre 500 et 20 000 u/L, de préférence entre 2 000 et 8 000 u/L et plus préférentiellement encore environ 5 000 u/L.

Outre les deux buts que se propose d'atteindre la susdite composition, elle peut également permettre de détruire les flores compétitives du microorganisme recherché si une telle démarche s'impose en fonction de l'échantillon ou dudit microorganisme. Ainsi, la composition conforme à l'invention peut comprendre en outre au moins un agent antimicrobien.

Par agent « antimicrobien » ou « agent sélectif », on entend tout composé capable de détruire ou inhiber les flores compétitives du microorganisme recherché. Il est à souligner que ledit agent antimicrobien ou sélectif peut être un antibiotique.

Bien évidemment, la formulation antimicrobienne ainsi utilisée (comprenant au moins un agent antimicrobien) est spécifique du microorganisme recherché.

En effet, une des étapes ultérieures du procédé conforme à l'invention consiste en la détection d'au moins une activité spécifique du microorganisme recherché par fluorescence et par conséquent, ladite fluorescence doit être limitée à l'activité recherchée.

De plus, les agents antimicrobiens peuvent jouer un rôle sur la modification de l'intégrité membranaire des microorganismes ciblés et donc sur des paramètres mesurables comme le potentiel membranaire, le potentiel d'oxydoréduction ou les activités deshydrogénases. Par exemple, les agents antimicrobiens utilisés pour sélectionner des *Listeria* sont composés d'au moins la polymixine B, l'ofloxacine, l'amphotéricine B, la 5-fluorocytosine et le chlorure de lithium. Les agents sélectifs de *Salmonella* sont constitués de vert brillant et de sulfapyridine. Les agents sélectifs de *Staphylococcus aureus* comprennent de la glycine, du chlorure de lithium et de la déferoxamine. La sélection des *Escherichia coli* se fait par les sels biliaires. Les agents sélectifs de *Mycobacterium avium paratuberculosis* comprennent de la vancomycine hydrochloride et l'acide nalidixique. Les antibiotiques sélectifs de *Legionella pneumophila* sont constitués du mélange polymixine B, vancomycine, cycloheximide.

Par «conditionnement» du microorganisme recherché dans un échantillon selon le procédé de l'invention, on entend « induction » ou « activation » d'au moins une activité ou propriété spécifique dudit microorganisme. Cette étape est importante et est destinée à permettre la détection de l'activité ou de la propriété spécifique en question par un marquage fluorescent.

Par exemple, le conditionnement du microorganisme recherché peut consister en l'induction d'une activité enzymatique caractéristique du microorganisme recherché et il résulte de l'ajout au milieu d'enrichissement du microorganisme d'un substrat non fluorescent spécifique de ladite enzyme.

Il est à souligner que le conditionnement peut comprendre l'induction d'une ou de plusieurs activité(s) enzymatique(s).

Les substrats non fluorescents spécifiques des enzymes recherchées sont par exemple : l'isopropyl-β-D-thiogalactopyranoside, le mélibiose- méthyl-β-D-galactopyranoside pour activer la β-D-galactosidase, par exemple le méthyl-α-D-galactopyranoside pour induire la galactosidase, le p-nitrophényl-β-D-glucuronide, l'isopropyl-β-D-glucuronide, ou le méthyl-β-D-glucuronide sodium pour induire la glucuronidase, le méthyl-β-D-glucoside, la cellobiose et la salicine pour induire la β-Dglucosidase, le méthyl-caprylate pour induire la caprylate estérase, le 4-nitrophényl-palmitate, le méthyl-palmitate pour induire la palmitate estérase, l'acide 2-méthyl-propionique pour induire la propionate estérase.

Selon un mode préférentiel de mise en oeuvre de la présente invention et dans le cas où le conditionnement consiste en l'induction d'au moins une activité enzymatique spécifique du microorganisme recherché, les étapes a) et b) du procédé de l'invention peuvent être réalisées simultanément. La sensibilité et la rapidité de l'analyse peuvent s'en trouver améliorées. Dans ce cas, la durée totale de ces deux phases réalisées simultanément est comprise entre 6 h et 48 h, de préférence 12 h et 24 h et plus préférentiellement encore entre 15 et 18h.

Dans le cas où le microorganisme recherché dans l'échantillon est une bactérie Gram⁺, l'étape de conditionnement peut également comprendre une étape d'induction d'au moins un antigène de surface caractéristique ou spécifique dudit microorganisme comprenant l'ajout au milieu d'enrichissement du microorganisme d'un extrait de levure à une concentration comprise entre 5 et 50 g/L, de préférence entre 10 et 20 g/L et plus préférentiellement encore environ 10 g/L.

L'induction d'au moins un antigène de surface, dans le cas où le microorganisme recherché est une bactérie Gram⁺, en plus de l'induction enzymatique, s'explique par le fait qu'il est impossible, selon l'art antérieur, de réaliser un tri immunomagnétique à l'aide d'anticorps dirigés contre des antigènes de la membrane et de la paroi cellulaire. Sous des conditions classiques de culture, les bactéries à Gram⁺ synthétisent une capsule exopolysaccharidique pour se protéger d'une agression extérieure, ce qui masque les antigènes de la paroi cellulaire et de la membrane, les rendant inaccessibles à des anticorps. C'est la raison pour laquelle, selon un mode préférentiel de mise en oeuvre de la présente invention, l'inhibition de la synthèse de cette capsule est réalisée par l'ajout au milieu d'enrichissement d'un extrait de levure, activant ainsi l'expression d'antigènes de membrane et de la paroi rendus ainsi accessibles à des anticorps.

L'étape de concentration immunomagnétique du microorganisme recherché est réalisée à partir du milieu de conditionnement. La concentration immunomagnétique consiste, par le biais d'une réaction antigène-anticorps, à concentrer ledit microorganisme à partir du milieu de conditionnement.

L'anticorps mis en contact avec le microorganisme recherché est dirigé vers un antigène spécifique dudit microorganisme tout en étant greffé sur une bille magnétique. Ensuite, les complexes billes-anticorps-microorganismes sont séparés du milieu et le microorganisme est ensuite séparé du reste du complexe.

Cette étape de concentration immunomagnétique peut être réalisée avec les billes Dynabeads^{®} selon les recommandations du fournisseur, à l'exception du volume d'échantillon qui est doublé. La réaction peut également être réalisée avec les billes liées de façon covalente à des anticorps (secondaires) dirigés contre les anticorps (primaires) de lapin (Dynabeads MP-280 sheep anti-rabbit IgG).

A titre d'exemple, le protocole mis en oeuvre peut être le suivant : un volume de 1 à 50 ml d'échantillon, de préférence 1 à 5 ml, préférentiellement 2 ml, est mis en contact pendant 30 min à température ambiante avec 1 à 250 µg, de préférence 1 à 50 µg, préférentiellement de 10 µg d'anticorps dirigé contre la bactérie recherchée. Une quantité de billes Dynabeads (*Listeria, Salmonella...*) comprise entre 1.10⁶ et 250.10⁶, de préférence entre 10.10⁶ et 20.10⁶, préférentiellement 10.10⁶, lavées en PBS (14190-094, Invitrogen S.a.r.l) / BSA (ID-BIO Sa) 0,1% au préalable, est ajoutée et l'ensemble est incubé 30 min à température ambiante sous agitation douce et régulière. A l'aide d'un concentrateur de particules magnétique (MPC-E, Dynal Biotech SA ou tout autre appareil de tri magnétique), les billes sont récupérées sur la paroi des microtubes par élimination du liquide résiduel. Elles sont ensuite lavées dans un volume de PBS et récupérées à nouveau à l'aide du concentrateur de particules.

A l'issue de la réaction d'immuno-concentration, les billes sont reprises dans 225 µl de PBS. Les bactéries sont ensuite décrochées des billes. Le concentrateur magnétique sert ensuite à éliminer les billes et le surnageant de décrochage contenant les bactéries concentrées est récupéré dans un tube de 5 ml pour subir la réaction de marquage fluorescent.

L'étape d'immuno-concentration, outre le fait de concentrer les bactéries d'intérêt et donc d'augmenter d'un facteur 10 la sensibilité de détection, permet d'éliminer les bactéries ayant pu se développer dans le milieu d'enrichissement et/ou conditionnement malgré les éléments sélectifs.

Selon un autre mode de réalisation de l'invention, l'anticorps greffé sur la bille magnétique est dirigé contre un anticorps lui-même dirigé contre un antigène spécifique du microorganisme recherché. En d'autres termes, une première réaction antigène-anticorps est réalisée par la mise en contact du microorganisme recherché avec un anticorps dit « primaire » dirigé contre un antigène spécifique de ce microorganisme et une seconde réaction antigène-anticorps est réalisée par la mise en contact de l'anticorps « primaire » avec un anticorps dit « secondaire » greffé sur une bille magnétique et dirigé contre ledit anticorps primaire.

Selon un mode de réalisation préféré de la présente invention, les billes magnétiques présentent un diamètre compris entre 1 et 20 µm, de préférence entre 2 et 8 µm. Plus préférentiellement encore, les billes mises en oeuvre dans les exemples ont, comme indiqué, un diamètre de 2,8 µm ou 4,5 µm.

La taille des billes mise en oeuvre dans la présente invention est importante car elle doit permettre l'extraction des bactéries de milieux complexes avec une grande efficacité. Dans l'art antérieur, des billes présentant un diamètre très inférieur à celui des billes de l'invention ont été utilisées (nanobilles) mais les Inventeurs ont constaté que celles-ci donnaient lieu à un bruit de fond beaucoup trop important ne permettant pas clairement de distinguer les microorganismes des autres éléments du milieu.

Le marquage fluorescent des microorganismes recherchés, de préférence obtenus après concentration immunomagnétique, est réalisé par l'ajout au milieu contenant lesdits microorganismes d'au moins un substrat comprenant une partie spécifique de l'activité enzymatique à révéler et une partie marqueur.

Par le choix de ces substrats, selon qu'il y ait utilisation ou non par le microorganisme, il est possible de différencier et de caractériser des espèces ou sous espèces d'un genre microbien donné.

Selon un mode de réalisation préférée de l'invention, la partie marqueur est constituée par un marqueur fluorogène excité à 488 nm choisi dans le groupe comprenant les xanthènes, les acridines, les phycobiliprotéines, la cyanine et l'esculine.

Parmi les xanthènes, on peut citer plus particulièrement la fluorescéine et les dérivés de fluorescéine dont des exemples sont donnés ci-après, ainsi que la rhodamine. Parmi les phycobiliprotéines, on peut citer tout particulièrement la phycoérythrine.

Dans le cas où plusieurs substrats sont mis en oeuvre, chacun d'eux peut comprendre une partie marqueur différente de la ou des autres partie(s) marqueur, de façon par exemple à exprimer plusieurs couleurs de fluorescence.

Dans le cadre de la présente invention, il est important que la transformation du substrat ait lieu à l'intérieur de la bactérie et à ce que le produit fluorescent soit retenu dans la cellule.

De façon alternative, une perméabilisation transitoire des bactéries peut être réalisée par l'ajout d'isopropanol à 15 % et une incubation de 15 min à 37°C.

Le choix des marqueurs fluorescents est basé sur leur capacité à être retenus à l'intérieur des cellules. Par exemple, les formes carboxylées, aminées ou pentafluorobenzoylaminées des fluorescéines sont particulièrement bien retenues dans les cellules, consécutivement à l'action des enzymes intracellulaires qui fixent ces produits fluorescents libérés dans les cellules (Haugland, 1995).

Selon un autre mode de réalisation de l'invention, la partie du substrat spécifique de l'activité enzymatique à révéler est constituée d'un acide gras, d'un ose, d'un phosphate, d'un peptide et/ou d'un sulfate.

Les acides gras sont caractérisés par une chaîne carbonnée de longueur comprise entre 2 et 20 atomes de carbones. Préférentiellement, le substrat est choisi dans le groupe comprenant la fluorescéine-di-caprylate, la fluorescéine-di-palmitate, la fluorescéine-di-dibutyrate, la fluorescéine-di-dicaproate, la fluorescéine-di-dilaurate, la fluorescéine-di-propionate, la 5(6)-carboxy-dichloro-fluorescéine-diacétate.

Les oses sont des pentoses ou des acides glucuroniques. Préférentiellement, le substrat est choisi dans le groupe comprenant la fluorescéine-di-β-D-galactopyranoside, le fluorescéine-α-D-galactopyranoside, la fluorescéine-di-β-D-glucopyranoside, le fluorescéine-di-β-D-mannopyranoside, le fluorescéine-di-β-D-fucopyranoside, le fluorescéine-di-β-D-N-acétylgalactosaminide, le fluorescéine-di-β-D-N-acétylglucosaminide, le fluorescéine-di-β-D-xyloside.

Préférentiellement, les substrats peptidiques sont des acides aminés enchaînés (de 1 à 10 acides aminés) et greffés à la Rhodamine 110.

Préférentiellement, les substrats phosphate et sulfate sont la fluorescéine-diphosphate et la fluorescéine-disulfate respectivement.

Préférentiellement, les substrats fluorescents permettant de détecter les activités estérases, osidases, phosphatases, peptidases ou sulfatases sont ajoutés aux milieux de conditionnement à une concentration comprise entre 0,1 µM et 1 mM, de préférence 1 µM à 100 µM.

A titre d'exemple, les substrats suivants sont particulièrement intéressants :
- le 5,6-carboxy-dichloro-fluorescéine-diacétate pour révéler la forte activité estérasique de *Listeria monocytogenes,* et les conditions acides du contenu intracellulaire de cette bactérie ;
- la 5-(pentafluorobenzoylamino)fluorescéine ou le fluorescéine-di-bêta-D-glucopyrannoside pour révéler l'activité bêta-D-glucoside de *Listeria monocytogenes,* ladite activité étant amplifiée chez *Listeria monocytogenes* par l'adjonction du substrat salicine préférentiellement dans le milieu de conditionnement à la concentration de 1 à10 mM ;
- la fluorescéine-dipropionate pour révéler l'activité propionate estérase de *Listeria monocytogenes ;* l'induction de cette enzyme est réalisée par l'ajout de l'acide 2-méthyl-propionique préférentiellement dans le milieu de conditionnement à la concentration de 1 à 10 mM ;
- la fluorescéine di-phosphate comme révélateur de l'activité phosphatase alcaline chez *Staphylococcus aureus,* une répression de l'enzyme chez les Staphylococcus non-aureus peut être réalisée par l'ajout de phosphate inorganique à 0,3% dans le milieu de conditionnement ;
- l'acide fluorescéine-di-bêta-D-glucuronique ou 5-(pentafluorobenzoylamino)fluorescéine-di-bêta-D-glucuronique comme révélateur de la glucuronidase chez *Escherichia coli ;* une induction de l'enzyme chez le microorganisme est réalisée par l'ajout de 4-nitrophényl-bêta-D-glucuronide de 1 à 10 mM dans le milieu de conditionnement ;
- la fluorescéine-di-palmitate ou la 5-(6) carboxyfluorescéine-di-palmitate comme révélateur de la palmitate estérase chez *Mycobacterium avium paratuberculosis,* une induction de l'enzyme chez le micro-organisme est réalisée par l'ajout de 4-nitrophényl palmitate ou méthyl-palmitate de 1 à 10 mM dans le milieu de conditionnement ;
- la fluorescéine-di-caprylate ou la 5-(6)carboxyfluorescéine-di-caprylate comme révélateur de la caprylate estérase chez *Salmonella,* une induction de l'enzyme chez le microorganisme est réalisée par l'ajout de méthyl-caprylate de 1 à 10 mM dans le milieu de conditionnement.

Les réactions enzymatiques peuvent se produire dans une gamme de température variant de 25 à 45°C selon les microorganismes, à l'abri de la lumière et pour une durée n'excédant pas 1 h. Sitôt la réaction de marquage achevée, les microorganismes sont stockés à 4°C pour limiter leur développement et surtout empêcher l'efflux de marqueur à l'extérieur du microorganisme.

Par ailleurs, il est intéressant de souligner que lorsque l'étape de conditionnement du procédé conforme à l'invention consiste en une étape d'induction d'au moins une activité enzymatique spécifique du microorganisme recherché, l'étape de concentration immunomagnétique peut avoir lieu avant l'étape de conditionnement du microorganisme en question, ou après l'étape de marquage fluorescent du microorganisme.

En d'autres termes, l'étape de conditionnement du microorganisme recherché peut avoir lieu dans un milieu comprenant ledit microorganisme préalablement concentré de façon immunomagnétique, et donc l'étape de marquage fluorescent interviendra après l'étape de conditionnement ; mais il est également possible d'envisager que l'étape de marquage fluorescent du microorganisme intervienne immédiatement après l'étape de conditionnement et que la concentration immunomagnétique du microorganisme n'intervienne qu'après ledit marquage fluorescent.

Selon un mode de réalisation préférée de l'invention, la détection et l'analyse de la fluorescence permettant la numération ou le comptage des microorganismes recherchés est réalisée par une technique choisie dans le groupe comprenant : la cytométrie en flux, la cytométrie sur filtre et la microscopie de fluorescence, la cytométrie en flux étant préférée.

La cytométrie en flux s'est en fait avérée particulièrement intéressante, en tant que dernière étape du procédé conforme à l'invention, afin de pouvoir rendre un résultat d'analyse fiable et rapide, c'est-à-dire en 24 h, voire 12 h.

La cytométrie en flux a connu récemment, en microbiologie, plusieurs développements : le dénombrement des microorganismes totaux en utilisant des marqueurs cellulaires non spécifiques, la détection et l'identification de microorganismes à l'aide de marqueurs spécifiques (immunoglobulines ou marqueurs génétiques) couplés à des fluorochromes (Robinson J. P., 1999).

De plus, cette technique s'applique parfaitement bien aux différents types d'échantillons mentionnés ci-dessus mais trouve également des applications dans le diagnostic vétérinaire et les analyses environnementales.

Chez l'animal contaminé par des microorganismes pathogènes, la présence de ces derniers peut être révélée de manière directe, ou indirecte. La voie directe consiste à détecter dans des biopsies ou des tissus, lesdits microorganismes par culture dans un milieu adapté (boîte de Pétri) ou à détecter par des méthodes ELISA des antigènes microbiens ou la recherche de séquences cibles dans l'ADN. La voie indirecte consiste en la détection des anticorps dirigés contre les antigènes microbiens qui sont produits en réponse à l'infection de l'animal par ces microorganismes. Le but est de diagnostiquer le plus précocement possible l'état sanitaire d'un animal, pour éviter que celui-ci ne contamine le reste du troupeau. Les méthodes d'analyses doivent donc être rapides et précoces dans le stade de développement de la maladie. Ainsi, il faut améliorer la détection des bactéries qui poussent très lentement dans un milieu de culture (exemple des mycobactéries) ou dont la propagation dans un cheptel est très rapide et passe parfois inaperçue au cours de ce que l'on appelle une phase asymptomatique (paratuberculose et maladie des muqueuses bovines).

Comme précédemment indiqué, les échantillons susceptibles d'être analysés par le procédé conforme à l'invention peuvent être d'origines très diverses mais également de consistance et/ou de nature très diverses. Ainsi, les caractéristiques physiques dudit échantillon peuvent nécessiter une étape préalable aux étapes a), b), c), d) et e) du procédé de l'invention. Plus particulièrement, il s'agit d'une étape de filtration de l'échantillon à analyser.

Dans le cas par exemple où l'échantillon à analyser est un échantillon solide, semi-solide ou constitué d'un liquide trouble, ladite filtration, appelée également broyage, est réalisée au moyen d'un filtre dont la porosité est comprise entre 20 et 150 microns, de préférence entre 30 et 100 microns et plus préférentiellement encore d'environ 63 microns.

A titre d'exemple, la filtration des échantillons solides peut être réalisée dans un sac plastique dans lequel est inséré sur toute sa surface un filtre pleine page en plastique présentant la porosité ci-dessus mentionnée.

Dans le cas où l'échantillon à analyser par le procédé conforme à l'invention est constitué d'un liquide clair, il peut être filtré sur une membrane présentant une porosité comprise entre 0,2 et 10 µm, de préférence 0,2 et 5 µm et plus préférentiellement encore entre 0,2 et 0,5 µm.

La présente invention a également pour objet un kit pour la mise en oeuvre du procédé ci-dessus décrit de détection et de comptage de microorganismes, ce kit comprenant :
- un milieu d'enrichissement sous une forme liquide ou déshydratée comprenant:
- une composition nutritive permettant la multiplication dudit microorganisme, et
- une composition de revivification sélective dudit microorganisme comprenant:
   - du pyruvate de sodium à une concentration comprise entre 1 à 20 g/L, de préférence entre 1 à 10 g/L,
   - du thiosulfate de sodium à une concentration comprise entre 0,5 et 5 g/L, de préférence entre 0,5 et 3 g/L,
   - de la catalase à une concentration comprise entre 500 et 20 000 u/L, de préférence entre 2 000 et 8 000 u/L,
   - un sac plastique doublé d'un filtre pleine-page présentant une porosité d'environ 63 µm,
   - des billes magnétiques telles que définies dans la revendication 8, un ou plusieurs substrats tels que définis dans la revendication 11 sous forme lyophylisée, des solvants appropriés.

La présente description sera mieux comprise à la lumière des exemples ci-après. Ces exemples ne précisent ni la nature, ni l'origine de l'échantillon à analyser. Ils identifient le microorganisme recherché et illustrent la détection de ce microorganime, étant entendu que le protocole décrit est le même quelque soit la nature ou l'origine de l'échantillon. Seule l'étape de broyage ou de filtration de l'échantillon, préalablement à l'étape d'enrichissement, n'est pas décrite.

### EXEMPLES

### Exemple 1 : Détection de Listeria monocytogenes

**Procédure pour activer l'enzyme bêta-D-glucosidase et les antigènes somatiques 1 et 4 de *Listeria monocytogenes,* et détection par cytométrie.**

*Listeria monocytogenes* est un bacille Gram positif, catalase+, oxydase-, non sporulé, non encapsulé, pathogène pour l'homme chez qui il est responsable de la listériose, maladie pouvant provoquer des avortements, des septicémies et des méningo-encéphalites.

Il fait partie du genre des *Listeria* qui comprend 6 espèces (*monocytogenes, ivanovii, innocua, welshimeri, seeligeri et grayi*)*.* Les souches de *Listeria monocytogenes* sont divisées en 17 sérotypes basés sur les antigènes somatiques (de 1 à 15) et flagellaires (de A à E) dont 3 (1/2a, 1/2b et 4b) représentent 95 % des souches isolées et sont responsables des listérioses humaines (Swaminathan *et al.,* 1995).

Les caractéristiques de survie et de multiplication de cette bactérie expliquent sa large répartition. Elle peut se multiplier en l'absence d'oxygène, à des pH pouvant aller de 5,6 à 9,6 et à des températures comprises entre 1 et 45 °C; elle résiste au sel (jusqu'à 20 % pour certaines souches), au dessèchement et à la congélation. Enfin, elle peut former ou participer à des biofilms favorisant sa persistance malgré le nettoyage et la désinfection.

Capable de persister dans des conditions hostiles pour la plupart des autres germes, elle est cependant assez peu compétitive et est inhibée par les flores microbiennes complexes. Dans les matrices alimentaires, les *Listeria monocytogenes* sont parfois en très faible nombre associées à une flore complexe composée souvent de streptocoques, entérocoques, microcoques, *Bacillus sp., Escherichia coli, Pseudomonas aeruginosa* et *Proteus vulgaris* (Kramer et Jones, 1969).

La transmission des *Listeria* se fait essentiellement par des aliments contaminés. La dose infectante à l'origine de cas cliniques est supérieure à 100 germes par g ou par ml.

L'enzyme β-D-glucosidase est présente chez toutes les espèces de *Listéria,* mais seule l'espèce *monocytogenes* possède la phosphatidylinositol phospholipase C (Notermans *et al.,* 1991). L'activité phosphatidylinositol phospholipase C est aussi retrouvée chez d'autres espèces microbiennes comme Bacillus.

Les caractéristiques des *Listeria monocytogenes* concernant la croissance, la sérologie et la biochimie sont à l'origine de la définition du milieu d'enrichissement utilisé pour revivifier et multiplier les *Listeria monocytogenes* à partir d'échantillons complexes tout en inhibant les bactéries compétitives, pour stimuler la production des antigènes somatiques 1 et 4 et pour induire l'expression de l'enzyme β-D-glucosidase qui servira à la détection des bactéries par l'utilisation d'un substrat fluorescent.

Le bouillon sélectif utilisé couramment pour *Listeria* est le Fraser 1/2. La composition de ce milieu a servi de base pour notre bouillon en ce qui concerne la base d'enrichissement non sélective. A cette base est ajoutée un supplément de revivification qui permettent de combler une carence du Fraser 1/2 : mettre en évidence des bactéries stressées. D'autre part un suplément d'induction d'activité β-D-glucosidase est ajouté. Le supplément sélectif du Fraser 1/2 a été modifié pour être efficace sur l'ensemble des produits complexes sur lesquels nous avons effectué la procédure complète de détection des *Listeria monocytogenes.*

Une autre réalisation particulière pour *Listeria monocytogenes* consiste à activer l'enzyme propionate estérase par l'ajout d'acide propionique à 2 mM dans le milieu de conditionnement. Cette activité est absente chez les entérocoques, ou autres coques à Gram positif, qui peuvent se développer de manière non spécifique dans le bouillon.

### 1) Compositions

### a. Base d'enrichissement non sélectif.

Tryptone peptone 5 à 15 g/L, concentration préférée 10g/L, , Extrait de levure 5 à 15 g/L, concentration préférée 10g/L, Na₂HPO₄ 9,6 g/L, KH₂PO₄ 1,35 g/L, N_{A}Cl 10 à 30 g/L, concentration préférée 20g/L.

La tryptone peptone et l'extrait de levure sont la source d'azote, de carbone, de vitamines et de minéraux. Ils permettent la production des antigènes. Les sels de Na₂HPO₄ et KH₂PO₄ permettent d'ajuster le pH du milieu à 7 +/- 0,2, pH de croissance optimum pour listéria et de tamponner le milieu au cours de l'enrichissement. La forte concentration en NaCl a pour but d'inhiber les entérocoques, flore souvent associée aux listéria qui présentent des caractéristiques communes avec elles notamment l'activité β-D-glucosidase.

### b. Supplément de revivification.

Pyruvate de Na 1 à 10 g/L, concentration préférée 5 g/L, Thioglycolate de Na, 0,5 à 5 g/L, concentration préférée 2,5 g/L, Catalase 5 000 u/L

Le pyruvate et le thioglycolate de Na est ajouté pour participer à la stimulation du métabolisme des organismes stressés. La catalase intervient dans l'élimination des espèces réactives de l'oxygène, toxiques pour les microorganismes et potentiellement présents dans les aliments.

### c. Complément d'induction de l'activité β-D-glucosidase.

Salicine de 1 à 20 mM, de préférence 2 mM.

A l'image de certaines β-D-glucosidases de champignons (Birk *et al.,* 1997 ; Perez-Pons J.A. 1995 ; Venturi et al. 2002) ou de bactéries (Yang *et al.,* 1996), la β-D-glucosidase de *Listeria monocytogenes* est inductible. En absence d'inducteur pendant la phase d'enrichissement l'activité enzymatique est indétectable.

La salicine est un substrat de la β-D-glucosidase ; sa présence dans le bouillon d'enrichissement permet l'induction de l'expression de cette enzyme et sa détection par cytométrie en flux. (cellobiose ou méthyl-β-Dglucoside peuvent également être utilisées).

### d. Complément d'induction de l'activité propionate estérase

Acide 2-méthyl-propionique, de 1 à 20 mM, concentration préférée 2 mM.

La présence de l'acide 2-méthyl-propionique dans le bouillon de conditionnement permet d'induire l'activité propionate estérase de *Listeria monocytogenes,* en vue de sa détection par un marquage fluorescent et cytométrie en flux.

### e. Complément sélectif.

Polymixine B 5mg/L, Ofloxacine 1mg/L, Amphotéricine B 2 mg/L, 5-Fluorocytosine 4 mg/L et éventuellement du chlorure de lithium 9 g/L.

La polymixine B est un antibiotique bactéricide actif sur les bactéries Gram négatif, agissant notamment sur *Escherichia coli* et *Pseudomonas aeruginosa*. L'ofloxacine complète l'action de la polymixine B sur les bactéries Gram négatif avec une activité bactéricide entre autre sur *Proteus vulgaris.* Dans le Fraser 1/2, c'est le chlorure de lithium associé à l'acide nalidixique qui inhibent les bactéries à Gram négatif; ils n'ont aucun effet sur *Pseudomonas* et *Proteus.* L'activité de l'ofloxacine s'étend également sur certaines espèces de streptocoques et staphylocoques. L'amphotéricine B est fongistatique ou fongicide selon les souches et la 5-fluorocytosine est fongicide. Il n'y a pas de fongicide dans la composition du Fraser 1/2. Certains produits, notamment les saucisses chipolatas sont chargées en levures qui interfèrent dans la réaction de détection par la beta-D-glucosidase. L'acriflavine inhibant les coques Gram positif n'est pas ajouté dans le milieu de conditionnement, car il rend les éléments du milieu fluorescents ce qui donne un bruit de fond sur les cytogrammes interférant dans la lecture de ceux-ci. Les coques les plus gênantes pour notre procédure sont celles qui possèdent l'activité β-D-glucosidase, ce sont les entérocoques dont la pousse est inhibée par la forte concentration en NaCl du bouillon. L'élimination des faux positifs résultant du marquage de l'activité glucosidase dans les coques à Gram positif peut se faire par une révélation de l'activité propionate-estérase qui est absente chez les coques et activable chez *Listeria monocytogenes.*

L'échantillon est dilué dans le bouillon sans le supplément sélectif à raison d'un facteur 5 à 10 (p/p). La température d'incubation à utiliser est de 30°C et le temps d'incubation de 12h. Au bout de 4h de revivification, le supplément sélectif est ajouté, suit la phase d'enrichissement sélectif de 8 h à 30°C.

### 2) Immunoconcentration

La réaction est réalisée avec des billes magnétiques (par exemple les Dynabeads^{®}-Listeria Dynal Biotech, diamètre de 2,8 µm) selon les recommandations du fournisseur, mais cet anticorps n'est pas spécifique de l'espèce *monocytogenes.*

Différents anticorps peuvent être utilisés sur les billes :
- un anticorps spécifique « Listeria O antiserum poly Serotypes 1,4 » (référence 223021, BD Diagnostics Systems) spécifique des souches de *Listéria monocytogenes* responsables des listérioses humaines et qui expriment les antigènes de type 1 et 4,
- un sérum spécifique de *Listeria monocytogenes* développé en inoculant des bactéries inactivées à un lapin, lesdites bactéries ayant au préalable été cultivées dans le bouillon de conditionnement.

Cette étape peut être réalisée indirectement avec une première réaction en présence d'un anticorps primaire (un des deux cités ci-dessus) non greffé sur des billes puis une immunoconcentration réalisée avec des billes recouvertes d'un anticorps secondaire reconnaissant l'anticorps primaire (par exemple Dynabeads M-280 sheep anti-rabbit IgG, diamètre 2,8 µm, référence 112-01, Dynal Biotech).

### 3) Marquage enzymatique

### a. Détection d'une activité estérasique.

Le substrat 5,6-carboxy-dichloro-fluorescéine-diacétate (référence 21884, Fluka Sigma-Aldrich Chimie S.a.r.l) est préparé dans du diméthyl formamide à la concentration de 10 mM, puis dilué à 10 µM dans du PBS. La réaction de marquage se fait avec 25 µl de la dilution ajoutés au surnageant de décrochage de l'immunoconcentration pendant 15 min à 37°C. Au cours de la réaction, la 5(6)-carboxy-dichloro-fluorescein est libérée dans le microorganisme et devient fluorescente. Cette molécule à la particularité d'émettre une fluorescence verte dans des gammes de pH acide (Nedergaard *et al.,* 1990).

Ce marqueur présente une spécificité vis à vis de *Listeria monocytogenes* par rapport à de nombreuses souches lorsqu'il est utilisé à la concentration de 1 µM finale (*Salmonella, Staphylococcus aureus, Escherichia coli, Proteus vulgaris, Bacillus sp., Yersinia* alvei). Il s'agit sans doute d'une particularité de cette bactérie, liée au fait qu'elle possède une activité estérase plus forte que pour les autres espèces de *Listeria* ; particularité qui se double d'un pH intracellulaire plus acide (nos observations). S'il est choisi pour faire la détection des *Listéria* par cytométrie en flux, la salicine n'est pas utile pour le milieu d'enrichissement.

### b. Détection de l'activité β-D-glucosidase.

Le substrat fluorescéine di-β-D-glucopyranoside ou pentafluorobenzoylaminofluorescéine-di-β-D-glucopyranoside est préparé à 20 mM dans l'eau selon la procédure recommandée par Molecular probes. Il est dilué à 4 mM dans H₂O avant utilisation à 500 µM final.

Dans le cas où le substrat est le fluorescéine-di-β-D-glucopyranoside, les bactéries récupérées par immunoconcentration sont perméabilisées par addition de 15 µl d'isopropanol suivie d'une courte incubation de 5 minutes à 37°C avant l'addition du substrat.

Dans tous les cas, la réaction enzymatique est effectuée à 37°C pendant 1 h, puis placée à 4°C immédiatement.

### c. Détection de l'activité propionate estérase.

Le substrat fluorescéine-dipropionate est préparé à une concentration de 10 mM dans du DMSO 10mM. Il est ajouté à l'échantillon de manière à ce que la concentration finale du substrat soit 100 µM finale. L'échantillon est alors incubé 15 min à 37°C, puis placé à 4 °C immédiatement dans l'attente de l'analyse.

### Exemple 2 : Détection de Salmonella.

**Procédure pour activer l'enzyme caprylate estérase, l'expression des antigènes de surface de *Salmonella,* et détection par cytométrie.**

Daniel E. Salmon, vétérinaire aux Etats-Unis, a découvert la première souche de salmonelle en 1885. On en connaît aujourd'hui 2 213 et la liste n'est pas close. Les salmonelles sont des bacilles à Gram négatifs anaérobies falcultatifs, (oxydase (-), catalase (+), asporulés), qui réduisent les nitrites en nitrates et fermentent le glucose. Le genre *Salmonella* comprend plus de 2500 sérotypes dont une cinquantaine sont vraiment importants dans nos régions. La formule antigénique est basée sur la nature des antigènes somatiques O, flagellaires H, ou capsulaire K (l'antigène Vi). Cet antigène capsulaire masque l'antigène O et est révélé après chauffage. Les antigènes O correspondent au lipopolysaccharide du LPS. Certaines souches peuvent perdre tout ou partie de ce pouvoir antigénique : formes R ou T. L'antigène H est soumis à un phénomène de variation de phase : il peut se trouver sous deux formes dénommées 1 et 2.

Première cause de toxi-infection alimentaire collective (TIAC), la salmonellose représente à elle seule environ les 2/3 des TIAC. Ces dernières années ont vu une augmentation spectaculaire, en incidence et en gravité, des cas de salmonelloses humaines. Par rapport à 1980, certains pays ont connu une multiplication de l'incidence par 20 sur les dix ou quinze dernières années. Lorsqu'on a étudié de manière plus approfondie les épidémies, on a découvert que la majorité des cas avaient pour origine des souches, ou plus exactement des sérotypes de *Salmonella enteritidis* et *Salmonella typhimurium.* La situation s'est encore aggravée depuis le début des années 1990 : des souches de *S*. *typhimurium,* résistantes à de nombreux antibiotiques, sont apparues et sont susceptibles de devenir un grave problème de santé publique.

L'être humain contracte surtout les salmonelloses en consommant de la nourriture contaminée d'origine animale, crue ou pas assez cuite (principalement de la viande, des volailles, des oeufs et du lait), bien qu'un grand nombre d'autres aliments aient été impliqués dans la transmission. L'agent causal passe dans la chaîne alimentaire à partir de la production primaire ou lors d'une contamination croisée avec des aliments à domicile ou dans des services de restauration collective ou des institutions comme les hôpitaux. La bactérie se multiplie encore à + 5°C/+ 12°C voire à une température inférieure à + 5°C. Elle survit bien à la congélation, la salaison et la déshydratation.

La transmission de personne à personne est rare dans les pays développés mais se produit parfois, notamment dans les institutions comme les services de soins néonatals ou les résidences pour personnes âgées.

Sur le plan épidémiologique, sont classés en fonction de leur adaptation à l'hôte humain ou animal et les antigènes somatiques O, trois groupes de salmonelles :
- le groupe B (51,8 % des cas), par exemple *Salmonella typhimurium* et *Salmonella paratyphi B,* provoque des fièvres entériques uniquement chez l'être humain et les primates supérieurs. Il se caractérise par des souches qui expriment l'antigène somatique 4 systématiquement et les antigènes 1, 5, 12 et 27 selon les sous-espèces concernées,
- le groupe D (19,1 % des cas) donne des maladies chez certains animaux, *Salmonella dublin* chez les bovidés, *Salmonella cholerae-suis* chez le porc et *Salmonella enteritidis,* mais rarement chez l'homme. Toutefois, lorsque l'affection touche l'être humain, elle prend souvent un caractère invasif et peut être mortelle. Les salmonelles composant ce groupe expriment toujours l'antigène 9 et selon les sous-espèces les antigènes 1 et 12,
- les bactéries du groupe C (20, 3 % des cas) expriment les antigènes 6,7 ; 6,8 ou 8, celles du groupe E (6,2 % des cas) les antigènes 3,10 ; 3,15 ; 1,3,19 ; celle du groupe G (1,2 %) les antigènes (13,22; 13,23); celles du groupe K l'antigène 18 et celle du groupe A (0,24 % des cas) *Salmonella paratyphi A* les antigènes 1,2.

La dose infectante de Salmonelles est de l'ordre de 100 000 germes. Les symptômes consécutifs à l'ingestion d'une telle dose sont des fièvres typhoïdes, lorsque les serovars incriminés sont *Salmonella typhi* et *paratyphi* A, B et C (House *et al.* 2001). Après une durée d'incubation variable de 1 à 25 jours, la maladie se déclenche avec des syndromes digestifs (diarrhées, douleurs abdominales, vomissements) avant d'entrer dans une phase septicémique lymphatique avec fièvre et torpeur. Cette manifestation est due à l'action au niveau cérébral d'une endotoxine neurotrope, le LipoPolySaccharide (LPS). Des sérovars moins pathogènes comme *Salmonella enteritidis, typhimurium, cholera suis, dublin* etc.. sont eux responsables de toxi-infections alimentaires moins sévères que l'on appelle salmonelloses.

Les entérobactéries pathogènes dont fait partie *Salmonella,* peuvent être recherchées directement sur des milieux sélectifs qui inhibent la croissance des bactéries Gram (+) et partiellement des coliformes, de *Proteus* et autres Gram (-). Ces milieux contiennent pour cela des extraits de bile, de désoxycholate de citrate, de sulfate ou de vert brillant en combinaisons diverses.

Le test de la β-galactosidase permet de distinguer entre les souches de salmonelles (β-Gal -) et celles de *Shigella* (β-Gal +). Ces deux germes bactériens possédant des conditions de culture très proches, sont fréquemment rencontrés conjointement. Une caractéristique particulière de *Salmonella* est sa capacité a métaboliser l'acide caprylique (activité C8 estérase). La mesure de l'activité caprylate estérase est utilisée dans un test utilisant la 4-methylumbelliferone qui fluoresce lorsqu'elle est excitée par des UV (*Humbert et al.,* 1989 ; Olsson *et al.* 1991). Certains milieux de culture utilisent une combinaison de 2 substrats chromogènes car les Salmonelles sont β-galactosidase négatives et glucuronate positives (milieux de culture SMID).

### 1) Compositions

### a. Base d'enrichissement non seléctif.

Tryptone peptone 5 à 15 g/L, concentration préférée 10 g/L, NaCl, 10 à 30 g/L, concentration préférée 20 g/L, Extrait de levure 5 à 15 g/L, concentration préférée 10 g/L, D-Glucose 0,5 à 3 g/L, concentration préférée 1 g/L, Na₂HPO₄ 5 à 15 g/L, concentration préférée 9,6 g/L, KH₂PO₄ 0,5 à 3 g/L, concentration préférée 1,35 g/L.

La tryptone peptone et l'extrait de levure sont la source d'azote, de carbone, de vitamines et de minéraux. Ils permettent la production des antigènes. Les sels de Na₂HPO₄ et KH₂PO₄ permettent d'ajuster le pH du milieu à 7 ± 0,2, pH optimal de croissance pour *Salmonella,* et de tamponner le milieu au cours de l'enrichissement. La forte concentration en NaCl a pour but d'inhiber les entérocoques.

### b. Suppléments de revivification.

Pyruvate de Na 1 à 10 g/L, concentration préférée 5 g/L, Thioglycolate de Na , 0,5 à 5 g/L, concentration préférée 2,5 g/L, Catalase 5 000 u/L.

Le pyruvate et le thioglycolate de Na est ajouté pour participer à la stimulation du métabolisme des organismes stressés (Bailey and Cox, 1992). La catalase intervient dans l'élimination des espèces réactives de l'oxygène, toxiques pour les microorganismes et potentiellement présents dans les aliments. La température d'incubation est comprise entre 20 et 40°C, de préférence 35°C.

### c. Complément d'induction de l'activité caprylate estérase.

Le 4-nitrophényl caprylate est un substrat de la caprylate estérase; sa présence dans le bouillon d'enrichissement permet l'induction de l'expression de cette enzyme et sa détection par cytométrie en flux.

### d. Complément sélectif.

Vert brillant 1 à 10 mg/L, concentration préférée 5 mg/L, pour inhiber la croissance des bactéries Gram +, Sulfapyridine à 1 g/L pour inhiber le développement des *E. coli* notamment.

Ces compléments ne génèrent pas d'autofluorescence contrairement aux compléments utilisés traditionnellement (par exemple sodium taurocholate ou autres sels billiaires, rouge de phénol, citrates ferriques...) qui sont responsables soit d'une forte autofluorescence du milieu, soit de la formation d'incrustations (corps réfringents) dans les bactéries. La température d'incubation, en présence des suppléments d'induction de l'activité caprylate estérase et des suppléments sélectifs, est de 20 à 40°C (de préférence 35°C). La durée d'incubation en présence de ces suppléments est de 6 à 15 heures en fonction du seuil de détection recherché.

### 2) Immunoconcentration

La réaction est réalisée avec des billes magnétiques (par exemple les Dynabeads^{®}-Salmonella, diamètre 2,8 µm, Dynal Biotech) selon les recommandations du fournisseur.

Différents anticorps sont utilisés sur les billes :
- un anticorps spécifique de Salmonella du commerce (par exemple référence 2302-50, BD Diagnostic Systems) pour un marquage direct. Cet anticorps dirigé contre les antigènes 1, 4, 5 et 12, permet de détecter les *Salmonella* du groupe D,
- un sérum spécifique de *Salmonella* développé en inoculant des bactéries inactivées à un lapin, lesdites bactéries ayant au préalable été cultivées dans le bouillon de conditionnement.

Cette étape peut être réalisée indirectement avec une première réaction en présence d'un anticorps primaire (un des deux cités ci-dessus) non greffé sur des billes puis une immuno-concentration réalisée avec des billes recouvertes d'un anticorps secondaire reconnaissant l'anticorps primaire (par exemple Dynabeads M-280 sheep anti-rabbit IgG, diamètre 2,8 µm, référence 112-01, Dynal Biotech).

### 3) Marquage enzymatique : détection de l'activité caprylate esterase.

Le substrat préférentiellement utilisé est la fluorescéine-dicaprylate. Une solution mère est préparée dans de l'acétone à une concentration de 10 à 400 µM. La concentration finale utilisée est de 1 à 40 µM, de préférence 10 µM. L'incubation en présence du substrat se fait de préférence à 37°C pendant 30 minutes à 1 heure puis les microorganismes sont immédiatement placés à 4°C.

### Exemple 3 : détection spécifique de Staphylococcus aureus

**Procédure pour activer l'enzyme phosphatase, l'expression des antigènes de surface de *Staphylococcus aureus,* et détection par cytométrie en flux.**

*Staphylococcus aureus* appartient à la famille des micrococaceae. C'est une coque Gram positif, non sporulée, immobile, de diamètre moyen compris entre 0,8 et 1 µm en moyenne. Son métabolisme respiratoire est fermentatif. Il est catalase + et pour la majorité de ces souches coagulase +. Il est responsable de nombreuses maladies : septicémie, conjonctivite, endocardite et ostéomyélite (Sheagren J. N., 1984).

La protéine A et les composants de la paroi cellulaire spécifiques de *Staphylococcus aureus,* sont masqués lorsque les souches sont cultivées sur un milieu d'induction des exopolysaccharides. Ces polysaccharides constituent une capsule et sont produits par environ 90 % des souches de *Staphylococcus aureus.* 11 sérotypes capsulaires ont été décrits, mais la plupart des souches isolées appartiennent aux sérotypes 5 et 8 (Arbeit *et al.,* 1984; Sompolinsky *et al.,* 1985). L'expression des sérotypes CP5 et CP8 de *Staphylococcus aureus* est largement influencée par les conditions environnementales et les conditions de croissance bactériennes. La production de CP5 est inhibée par un fort taux d'extrait de levure Dassy et ses collaborateurs (1991) et Ouyang et ses collaborateurs (1999) montrent le même effet inhibiteur de l'extrait de levure sur la production de la microcapsule CP8.

Les anticorps produits contre *Staphylococcus aureus* sont soit capsulaires soit membranaires. Pour une détection immunologique de ces bactéries, il faut utiliser un mélange de ces deux types d'anticorps (comme cela est fait pour le test Pastorex Staph plus, Biorad) ou empêcher la production de la capsule exopolysaccharidique avant de procéder à la réaction de reconnaissance antigène-anticorps. C'est cette deuxième stratégie que nous avons choisi de développer, avec la mise au point d'une composition de revivification inhibant la production de la capsule polysaccharidique.

### 1) Compositions

### a. Base d'enrichissement non sélectif.

Peptone de viande : concentration préférée 8 g/L, peptone de caséine : concentration préférée 2 g/L, extrait de levure : concentration préférée 10 g/L, extrait de viande : concentration préférée 5 g/L. Les peptones de viande et de caséine et les extraits de levure et de viande sont la source d'azote, de carbone, de vitamines et de minéraux. Ils permettent la croissance de *Staphylococcus aureus* et l'inhibition de la formation de la capsule.

### b. Supplément de revivification.

Pyruvate de Na 1 à 10 g/L, concentration préférée 5 g/L, Thioglycolate de Na , 0,5 à 5 g/L, concentration préférée 2,5 g/L, Catalase 5 000 u/L.

Le pyruvate et le thioglycolate de Na est ajouté pour participer à la stimulation du métabolisme des organismes stressés (Bailey and Cox, 1992). La catalase intervient dans l'élimination des espèces réactives de l'oxygène, toxiques pour les microorganismes et potentiellement présents dans les aliments.

### c. Complément de répression de l'activité phosphatase alcaline.

Phosphate inorganique 0,3 %.

L'activité phosphatase est une des activités associées au pouvoir entérotoxinogènes dés germes, et est donc indiquée pour une détection de *Staphylococcus aureus.*

L'activité phosphatase alcaline est constitutive chez *Staphylococcus aureus* (Soro *et al.,* 1990). Cependant, étant réprimée par le phosphate chez certaines souches de *Staphylococcus* non aureus (Soro *et al.,* 1990), il est utile d'ajouter des ions phosphates dans le milieu.

### d. Complément sélectif

Glycine 12 g/L, LiCl 5 g/L, déferoxamine 0,05g/L

Le chlorure de lithium est un inhibiteur des Bactéries à Gram négatif. La glycine est un inhibiteur des bactéries à Gram positif et stimule la croissance des Staphylocoques. La déferoxamine inhibe la croissance de *Staphylococcus epidermis,* qui est le seul staphylocoque avec *S*. *aureus* à posséder une activité phosphatase.

### 2) Immunoconcentration

Différents anticorps peuvent être utilisés sur les billes :
- un sérum spécifique de *Staphylococcus aureus* du commerce (référence 50-L030, AGROBIO) pour une fixation directe de l'anticorps sur les antigènes membranaires,
- un anticorps spécifique de *Staphylococcus aureus* développé en inoculant des bactéries inactivées à un lapin, qui se sont développées dans le milieu de conditionnement.

Cette étape peut être réalisée indirectement avec une première réaction en présence d'un anticorps primaire (un des deux cités ci-dessus) non greffé sur des billes puis une immunoconcentration réalisée avec des billes recouvertes d'un anticorps secondaire reconnaissant l'anticorps primaire (par exemple Dynabeads M-280 sheep anti-rabbit IgG, diamètre 2,8 µm référence 112-01, Dynal Biotech).

### 3) Marquage enzymatique.

### a. Détection de l'activité phosphatase alcaline.

Le substrat fluorescéine-diphosphate (référence F-2999, Molecular probes) permet de détecter l'activité phosphatase alcaline. Il est préparé par dissolution dans du Tris-HCl 100 mM pH 8,0 à la concentration de 10 mM, puis dilué à 1 mM dans du PBS. La réaction de marquage se fait avec 25 µl de la dilution, ajoutés au surnageant de décrochage de l'immunoconcentration et à 125 µl de PBS pendant 15 min à 37°C. Au cours de la réaction, la fluorescein est libérée et devient fluorescente (excitation/émission 490/514 nm).

Ce marqueur présente une spécificité vis à vis de certaines espèces de Staphylocoques (*aureus* et *epidermis*) de *Micrococcus luteus,* de *Escherichia coli,* et de quelques espèces de Streptocoques. La combinaison du bouillon et de l'immuno-concentration permet d'éliminer les souches susceptibles d'être positives au marquage fluorescent. La deferoxamine inhibe la croissance de *Staphylococcus epidermis.*

### Example 4 : Détection de Escherichia coli

### Procédure pour activer l'enzyme glucuronidase, l'expression des antigènes de surface de Escherichia coli et la détection par cytométrie

Thomas ESCHERICH a découvert cette bactérie en 1855. De la famille des *Enterobacteriaceae, Escherichia coli* est un genre bactérien dans lequel on ne retrouve qu'une seule espèce ; mais il existe plus de 1000 types antigéniques. Ces sérotypes sont définis selon leurs antigènes somatiques O (171), capsulaires K (80) et flagellaires H (56). De plus, les antigènes K sont subdivisés en types A, B ou L. Le type B est rencontré exclusivement dans les souches associées aux diarrhées infantiles.

Assez courte (2 à 3 µm x 0,7 µm), on la trouve isolée ou groupée par 2 ou plus rarement en amas. Elle peut apparaître sous forme cocco-bacillaire ou filamenteuse dans les cultures âgées. Sa mobilité péritriche est très réduite, voire nulle (ex sérotype 0111). Sa culture est très facile avec une grande tolérance de variation de pH (pH optimum 7,5). La température optimale de croissance est de 37°C, mais elle pousse entre 15°C et 45°C et se multiplie encore à + 5°C. Elle résiste bien à la chaleur : incubée à 45°C, elle fermente le glucose, le mannitol et le lactose avec production importante de gaz. Elle résiste à l'acidité et à la congélation. Elle reste relativement sensible aux antibiotiques et comme toutes les entérobactéries elle réduit les nitrates en nitrites. Elle fermente le glucose et le lactose et irrégulièrement le saccharose et la salicine. La plupart possèdent une activité lysine décarboxylase.

*E. coli* est un germe habituel de la flore intestinale de tous les animaux, y compris les humains. C'est un commensal de l'intestin qui représente 80 % de la flore intestinale aérobie. Le germe se retrouve dans les matières fécales. De là, il se répand dans la nature : sol et eaux. Sa présence dans le milieu environnant signe toujours une contamination fécale. Sa mise en évidence dans l'eau constitue le principe des tests colorimétriques.

On retrouve des *Escherichia coli* principalement dans les viandes crues ou insuffisamment cuites (boeuf, volaille) et les matières fécales d'origine animale et humaine. L'opération de hachage des viandes constitue une source de contamination favorisée par ce germe. Normalement, *E. coli* a plutôt une fonction de suppression de bactéries nuisibles et permet de synthétiser de nombreuses vitamines, car seule une minorité de souches d'*E*. *coli* sont capables de causer des infections humaines.

Les *Escherichia coli* sont classés en plusieurs groupes ou pathovars en fonction de leur pathogénicité. Cette pathogénicité est presque toujours basée sur une capacité d'adhésion à différents récepteurs cellulaires. Les *Escherichia coli* appartenant à ces pathovars sont distingués sérologiquement par :
- des antigènes somatiques O : il existe 180 variétés dont une trentaine sont fréquemment rencontrées chez les souches pathogènes ECEP de type I (026, 055, 086, 0111, 0119, 0125-0128, 0142...), ECEP de type II (018, 044, 0112, 0114...), ECEI (028, 029, 0124, 0136, 0143, 0152...), ETEC (06, 08, 015, 020, 025...) ECEH (026, 0113, O l21, 0145, 0157...),
- des antigènes capsulaires K de nature polysaccharidique A ou B (une dizaine de types importants de B chez les ECEP ou de nature protéique L (de pili en particulier les antigènes CFA),
- des antigènes flagellaires H, importants pour les ECEH dont le sérotype le plus courant est O157 H7.

La plupart des souches pathogènes est caractérisée par un sérotype particulier de type OxKyHz.

Lorsque *E. coli* est responsable d'une diarrhée aiguë, on peut le classer selon quatre pathotypes; entéropathogène (EPEC), entéroenvahisseur (ou entéroinvasifs -EIEC), entérohémorragiques (EHEC) et entérotoxinogène (ETEC). Les souches se caractérisent par leur capacité de produire une entérotoxine dont l'action sur les entérocytes perturbe les fonctions d'absorption normalement assurées par la muqueuse intestinale. Ces microorganismes peuvent êtres présents dans certains aliments, comme le boeuf haché, et dans l'eau où ils trahissent une contamination fécale.

Il peut, s'il arrive à franchir les muqueuses intestinale (lésions de la paroi intestinale), devenir pathogène et peut déterminer des infections urinaires, biliaires, génitales appelées colibacilloses, et très exceptionnellement une septicémie. Il se comporte en pathogène opportuniste.

### 1) Compositions

### a. Base d'enrichissement non seléctif.

Tryptone peptone10 à 30 g/L, concentration préférée 20 g/L ; Extrait de levure 5 à 15 g/L, concentration préférée 10 g/L ; Nacl 1 à 10 g/L, concentration préférée 5 g/L ; D-Lactose 1 à 10 g/L, concentration préférée 5 g/L ; Na₂HPO₄ 5 à 15 g/L, concentration préférée 9,6 g/L, KH₂PO₄ 0,5 à 3 g/L, concentration préférée 1,35 g/L

La tryptone peptone et l'extrait de levure sont la source d'azote, de carbone, de vitamines et de minéraux. Ils permettent la production des antigènes. Les sels de Na₂HPO₄ et KH₂PO₄ permettent de tamponner le milieu.

### b. Suppléments de revivification.

Pyruvate de Na 1 à 10 g/L, concentration préférée 5 g/L, Thioglycolate de Na , 0,5 à 5 g/L, concentration préférée 2,5 g/L, Catalase 5 000 u/L

Le pyruvate et le thioglycolate de Na est ajouté pour participer à la stimulation du métabolisme des organismes stressés (Bailey and Cox, 1992). La catalase intervient dans l'élimination des espèces réactives de l'oxygène, toxiques pour les microorganismes et potentiellement présents dans les aliments.

### c. Complément d'induction de l'activité glucuronidase.

Le 4 nitrophényl ß,D-glucuronide est un substrat de la glucuronidase; sa présence dans le milieu de conditionnement permet l'induction de l'expression de cette enzyme et sa détection par cytométrie en flux. Il est ajouté au milieu de conditionnement dans les proportions variant de 0,5 à 5 mM, de préférence 1 mM final.

### d. Complément sélectif.

Sels biliaires 0,5 à 5 g/L, de préférence 1,5 g/L

Ils sont utilisés pour inhiber le développement des bactéries Gram +, particulièrement les bactéries sporulantes et les streptocoques fécaux, et pour stimuler la croissance *d'Escherichia coli.* La température d'incubation en présence des suppléments d'induction de l'activité glucuronidase et des suppléments sélectifs est de 20 à 50°C, de préférence 37°C.

La durée d'incubation en présence de ces suppléments est de 6 à 15 heures en fonction du seuil de détection recherché.

### 2) Immunoconcentration

Différents anticorps peuvent être utilisés et greffés sur les billes :
- un anticorps spécifique de *Escherichia. coli* du commerce pour un marquage direct,
- un anticorps spécifique de *Escherichia coli* développé en inoculant des bactéries inactivées à un lapin, lesdites bactéries ayant préalablement poussé dans le milieu de conditionnement.

Cette étape peut être réalisée indirectement avec une première réaction en présence d'un anticorps primaire (un des deux cités ci-dessus) non greffé sur des billes puis une immunoconcentration réalisée avec des billes recouvertes d'un anticorps secondaire reconnaissant l'anticorps primaire (par exemple Dynabeads M-280 sheep anti-rabbit IgG, diamètre 2,8 µm référence 112-01, Dynal Biotech).

### 3) Marquage enzymatique : détection de l'activité glucuronidase

Le substrat préférentiellement utilisé est l'acide fluorescéine-di-β-D-glucuronique ou encore plus préférentiellement l'acide pentafluorobenzoylamino-fluorescéine-di-β-D-glucuronique. Une solution mère est préparée à une concentration de 10 mM dans l'eau. Une solution de travail est préparée à 2 mM dans H₂O. La concentration finale utilisée est de 50 à 500 µM, de préférence 160µM.

L'incubation en présence du substrat se fait de préférence à 37°C pendant 30 minutes à 1 heure.

Dans le cas où le substrat utilisé est l'acide fluorescéine-di-β-D-glucuronique, les bactéries sont perméabilisées temporairement par addition de 15 µl d'isopropanol pendant 5 minutes à 37°C.

Dans tous les cas, les microorganismes fluorescents sont ensuite immédiatement placés à 4°C avant analyse.

### Exemple 5 : Détection de Mycobacterium avium paratuberculosis

**Procédure pour activer l'enzyme palmitate estérase et l'expression des antigènes de surface de *Mycobacterium avium paratuberculosis,* et détection par cytométrie en flux**

*Mycobacterium avium paratuberculosis* est une bactérie à Gram positif et alcoolo-résistante qui mesure de 0,5 à 1,5 µm. Elle forme des colonies blanches et rugueuses sur le milieu de culture Herrold's egg yolk (HEYM). Il s'agit d'un microorganisme au développement très lent : les colonies sur un milieu solide sont visibles après 3 à 4 mois de culture. La présence de suppléments nutritifs dans le milieu n'améliore pas sa vitesse de croissance (Cocito *et al.,* 1994).

Les mycobactéries sont notamment résistantes aux facteurs physiques et chimiques. *M. avium paratuberculosis* est apparemment l'une des bactéries les plus résistantes de cette famille, ce qui explique sa capacité à survivre si longtemps dans l'environnement. Certains facteurs sont susceptibles de diminuer son temps de survie dans l'environnement : la sécheresse, l'exposition au soleil, un pH supérieur à 7,0 et un terrain pauvre en fer.

*M. avium paratuberculosis* est proche phylogénétiquement d'autres espèces appartenant à la même famille : il présente une homologie de l'ADN supérieure à 99 % avec *M. avium avium.* Cette forte homologie génétique se traduit, en conséquence, par un nombre élevé d'antigènes communs. Cela représente un problème non négligeable lorsqu'on utilise des tests immunologiques directs pour détecter spécifiquement les souches de *M. avium paratuberculosis.*

Les techniques actuelles couramment utilisées par les laboratoires d'analyses pour la détection de la bactérie *Mycobacterium avium paratuberculosis* repose sur trois technologies différentes : la sérologie, la méthode PCR, la coproculture.

L'analyse sérologique est une méthode indirecte basée sur la détection des anticorps anti *Mycobacterium avium paratuberculosis ;* elle permet d'obtenir des résultats en 24 heures, mais l'absence d'antigènes spécifiques dirigés contre les anticorps anti-mycobacterium, rend ces tests moins sensibles que la coproculture (37 %).

La technique PCR est une analyse directe, rapide (24 heures), sensible, mais ne permettant pas de mesurer ni de différencier les bactéries mortes des vivantes. C'est un problème d'importance lorsque l'on veut analyser des produits alimentaires qui ont subit une stérilisation. En effet, il peut persister dans des produits ayant subis un stress physique important de l'ADN provenant de bactéries mortes. Une méthode de détection de l'ADN donnerait donc des résultats faussement positifs.

La coproculture est une méthode sensible, fiable, mais très longue : les résultats sont obtenus en 3 à 4 mois.

Le protocole présenté ci-dessous décrit une méthode d'analyse, à partir de fèces bovins, permettant de déterminer la présence de *Mycobacterium avium paratuberculosis* et de la quantifier dans un délai court (5 à 7 jours).

Par ailleurs, la quantité de bactéries pathogènes présentes dans les fèces varie de façon importante au cours du cycle de l'infection ; aussi, une étape de revivification / enrichissement de la bactérie est introduite afin d'améliorer la sensibilité de la détection, notamment lors des stades précoces de développement de l'infection à *Mycobacterium avium paratuberculosis.*

### 1) Compositions

### a) Base d'enrichissement non sélectif.

Infusion Coeur-Cerveau 37 g/L, Glycérol 2,7 %, Asparagine 2g/L; Tween 80 0,1 %, Mycobactine J 2ml (Synbiotics Corporation ; réf. AMCE), Sérum de veau foetal 10% (SVF ; Invitrogen). Ce milieu de conditionnement est une préparation permettant dans un premier temps de revitaliser les bactéries stressées et/ou ayant une activité métabolique réduite et dans un second temps, de favoriser sélectivement, la croissance de *Mycobacterium avium paratuberculosis* dans le milieu.

### b) Supplément de revivification.

Pyruvate de Na 1 à 10 g/L, concentration préférée 5 g/L, Thioglycolate de Na, 0,5 à 5 g/L, concentration préférée 2,5 g/L, Catalase 5 000 u/L.

Le pyruvate et le thioglycolate de Na est ajouté pour participer à la stimulation du métabolisme des organismes stressés (Bailey and Cox, 1992). La catalase intervient dans l'élimination des espèces réactives de l'oxygène, toxiques pour les microorganismes et potentiellement présents dans les aliments.

### c) Complément sélectif.

Acide nalidixique 50 mg/L, Vancomycine à 50 mg/L.

### d) Complément d'induction.

Le 4-nitrophényl palmitate concentration de 0,5 à 5 mM, concentration préférée 2 mM.

L'enzyme palmitate estérase est une enzyme inductible chez *Mycobacterium avium paratuberculosis.* Le 4-nitrophenyl-palmitate est un substrat de cette enzyme. Son addition dans le bouillon de conditionnent permet l'induction de l'expression de cette enzyme et sa détection par cytométrie en flux.

Les bactéries du *Mycobacterium avium paratuberculosis* présentes dans les fèces bovins, bien que possédant un potentiel infectieux important, ne sont pas aptes dans la plupart des cas à se développer rapidement dans un milieu approprié. Soit elles sont stressées par leur environnement, soit elles présentent une activité métabolique réduite, qui est à l'origine de leur croissance très lente. Le pyruvate de sodium utilisé dans le milieu MCD8 possède des propriétés de revivification en agissant probablement sur le cycle de Krebbs. Par ailleurs, il est admis qu'un milieu riche en acides gras, et particulièrement en acide palmitique et acide oléique (Tween 80), favorise le développement des microorganismes et particulièrement de *Mycobacterium avium paratuberculosis.* La mycobactine J, en agissant comme un chélateur d'ions Fe²⁺, favorise l'entrée de cet ion indispensable à la croissance du mycobactérium. Enfin, le développement de *Mycobacterium avium paratuberculosis* s'accompagne toujours d'une libération de molécules toxiques de peroxyde d'hydrogène (H₂O₂). La catalase, présente dans le milieu, permet d'éliminer ces produits issus du métabolisme cellulaire. Les deux antibiotiques, à large spectre, additionnés au milieu permettent de limiter le développement d'autres souches bactériennes initialement présentes dans les fèces. La phase d'enrichissement dans le milieu de conditionnement est préconisée pour une durée minimale de 5 jours ; de très bons résultats sont obtenus avec 7 jours d'incubation.

### 2) Immuno concentration

Différents anticorps peuvent être utilisés et greffés sur les billes :
- un anticorps spécifique de *Mycobacterium avium* du commerce pour un marquage directe (Biodesign International),
- un anticorps spécifique de *Mycobacterium avium paratuberculosis* développé en inoculant des bactéries inactivées à un lapin, lesdites bactéries ayant préalablement poussé dans le milieu de conditionnement.

Cette étape peut être réalisée indirectement avec une première réaction en présence d'un anticorps primaire (un des deux cités ci-dessus) non greffé sur des billes puis une immunoconcentration réalisée avec des billes recouvertes d'un anticorps secondaire reconnaissant l'anticorps primaire (par exemple Dynabeads M-450 Goat anti-mouse IgG, diamètre 4,5 µm Dynal Biotech).

### 3) Marquage enzymatique

A la suite de l'étape d'immuno-concentration magnétique, *Mycobacterium avium paratuberculosis* est révélé par un fluorochrome spécifique de son activité enzymatique globale. Ce marquage permet de mettre en évidence les bactéries vivantes, potentiellement infectieuses et virulentes.

### a. Détection de l'activité palmitate estérase.

Le substrat préférentiellement utilisé est la fluorescéine-di-palmitate. Une solution mère est préparée dans de l'acétone à une concentration de 10 à 400 mM. La concentration finale utilisée est de 1 à 40 µM, de préférence 10 µM. L'incubation en présence du substrat se fait de préférence à 37°C pendant 30 min à 1 heure. Les microorganismes fluorescents sont alors immédiatement placés à 4°C avant analyse

### Example 6 : Détection de Legionella pneumophila

*Legionella* est un bacille à Gram négatif, non sporulé, aérobie strict et mesurant de 0,2 à 0,5 µm. De forme coccobacillaire dans les tissus infectés ; il est présent sous une forme filamenteuse sur milieu artificiel, où il forme des colonies grisâtres et polymorphes. Le genre *Legionella* comprend actuellement 43 espèces et 64 sérogroupes. Leur habitat naturel est l'eau, et est couramment reconnu comme parasite intra-cellulaire des amibes. Responsable de pathologies chez l'homme (les légionelloses), *Legionella pneumophilla* 1 est le sérogroupe le plus fréquemment rencontré en pathologie (80 % des cas).

Les facteurs favorisant la multiplication de *Legionella* sont la température de l'eau, la présence de biofilms, la présence d'amibes et la stagnation de l'eau.

*Legionella* se caractérise notamment par une richesse en acides gras ramifiés, inhabituelle pour les germes à Gram négatif. Elle exige pour sa culture de la L-cystéine et du pyrophosphate ferrique, qui sont des facteurs de croissance indispensables, mais elle n'hydrolyse pas les sucres et ne pousse pas sur des milieux gélosés au sang. Enfin, elle résiste à des traitements thermiques et acides forts (100°C pendant 10 min ; HCL 0,2 M).

Sur milieu artificiel, l'incubation de *Legionella* est réalisée à 36°C pendant 10 jours, avec des lectures à 3, 7 et 10 jours ; sa croissance est favorisée par 2,5 % de CO₂.

L'identification de *Legionella* peut être réalisée de deux manières distinctes : un examen direct par un test d'immuno-agglutination au latex ou par immunofluorescence à l'aide d'anticorps polyclonaux et monoclonaux ; une culture sur milieu sélectif. L'examen direct présente comme avantage d'être hautement spécifique, permettant ainsi une identification précise du sérotype ; cependant, cette méthode reste peu sensible et nécessite un minimum de 10 000 germes/mL pour permettre d'obtenir un résultat. Ces techniques sont donc utilisées afin d'identifier les espèces et sérogroupes à la suite d'une culture sur milieu sélectif.

La culture sur milieu sélectif gélosé présente comme avantage d'être relativement sensible (seuil de détection minimum à 50 UFC/mL), mais ne permet d'obtenir qu'une présomption de présence de *Legionella.* Cette technique doit, obligatoirement, être complétée par des examens directs (immunofluorescence directe ou immuno-agglutination de billes de latex) ou par des techniques de biologie moléculaire (PCR). Ces deux techniques présentent par conséquent comme inconvénient majeur d'être longues : 10 jours avant d'obtenir un résultat négatif.

### 1) Compositions

### a) Base d'enrichissement non sélectif.

Extrait de levure 10 g/L, ACES 10 g/L, Alpha-cétoglutarate 1 g/L; Charbon activé 4 g/L ; pH 6,9 ± 0,2 ; L-cystéine 0,4 g/L ; Pyrophosphate ferrique 0,25 g/L.

### b) Supplément de revivification.

Pyruvate de Na 1 à 10 g/L, concentration préférée 5 g/L, Thioglycolate de Na , 0,5 à 5 g/L, concentration préférée 2,5 g/L, Catalase 5 000 u/L.

### c) Complément sélectif.

Polymixine B 5 mg/L, 2 mg de Vancomycine hydrochlorique 2 mg/L, Cycloheximide 16 mg/L.

Le milieu de conditionnement *Legionella* possède comme base de formulation les composants décrits par Pine *et al.* (1979); l'extrait de levure et le tampon ACES, la glycine et la L-cystéine sont les composants classiques des milieux de cultures permettant l'apport de facteurs de croissance, de sels minéraux et d'acides aminés indispensables au développement de *Legionella* ; l'α-cétoglutarate est connu comme un puissant activateur de la croissance de la bactérie.

Enfin, *Legionella pneumophila* produisant au cours de sa croissance une grande quantité de radicaux libres, ce qui est incompatible avec son développement, le charbon activé permet d'éliminer ces molécules toxiques. La durée d'incubation dans le milieu de conditionnement est de 24 à 72 heures, préférentiellement 48 heures, à une température d'incubation comprise entre 20 et 50°C, de préférence d'environ 37°C.

### 2) Immuno concentration.

Différents anticorps peuvent être utilisés et greffés sur les billes :
- un anticorps spécifique de *Legionella pneumophilla* du commerce pour un marquage direct (Abcam UK),
- un anticorps spécifique de *Legionella pneumophilla* développé en inoculant des bactéries inactivées à un lapin, lesdites bactéries ayant préalablement poussé dans le milieu de conditionnement.

Cette étape peut être réalisée indirectement avec une première réaction en présence d'un anticorps primaire (un des deux cités ci-dessus) non greffé sur des billes puis une immunoconcentration réalisée avec des billes recouvertes d'un anticorps secondaire reconnaissant l'anticorps primaire (par exemple Dynabeads M-450 Goat anti-mouse IgG, diamètre 4,5 µm, Dynal Biotech).

### 3) Marquage enzymatique

Rhodamine 123,200 mM

La Rhodamine 123 est un marqueur fluorescent du potentiel membranaire. Une altération de ce dernier entraîne une incorporation moindre de ce fluorochrome dans les cellules. La répartition de cette sonde est de 1 unité à l'extérieur de la cellule, 10 000 dans le compartiment cytoplasmique. Cette spécificité cellulaire alliée au mode d'incorporation (potentiel dépendant), fait que la fluorescence émise traduit l'activité globale de la cellule.

La Rhodamine 123, composante du milieu de conditionnement, va être incorporée au cours de la multiplication de *Legionella* à l'intérieur des microorganismes, et permettre ainsi d'obtenir des bactéries fluorescentes à la longueur d'onde d'émission optimale de 633 nm.

Par ailleurs, l'adjonction d'antibiotiques à larges spectres permet d'éliminer les contaminations éventuelles de l'échantillon par des bactéries souvent associées à *Legionella,* tels que *Staphyloccocus aureus* ou *Pseudomonas aeruginosa.* Ces antibiotiques, agissant en découplant le potentiel membranaire, induisent un faible ou une absence de marquage par la Rhodamine 123.

### REFERENCES

- Alvarez-Barrientos et al. (2000) Applications of flow cytometry to clinical microbiology. Clinical Microbiology Reviews. 13 : 167-195.
- Amann (1995) Fluorescently labelled rRNA-targetted oligonucleotide probes in the study of microbial ecology. Mol. Evol. 4 : 543-554.
- Arbeit et al. (1984) Predominance of two newly described capsular polysaccharide types among clinical isolated of Staphylococcus aureus. Diagn. Microbiol. Infect. Dis. 2 : 85-91.
- Bailey and Cox (1992).
- Barer et al. (1999) Bacterial viability and culturability. Advances in Microbial Physiology; 41 : 93-137.
- Birk et al. (1997) Synthesis of isopropyl-1-thio-beta-D-glucopyranoside (IPTGlc), an inducer of Aspergillus niger B1 beta-glucosidase production. Appl Biochem Biotechnol. 66 : 25-30.
- Breewer et al. (1995) Characterization of uptake and hydrolysis of fluorescein diacetate and carboxyfluorescein diacetate by intracellular esterase in Saccharomyces cerevisiae, which results in accumulation of fluorescent product. Appl. Envrion. Microbiol. 61 : 1614-1619.
- Cocito et al. (1994) Paratuberculosis. Clin. Microbiol. Rev. 7 : 328-345.
- Dassy et al. (1991) Production of type 5 capsular polysaccharide by Staphylococcus aureus grown in a semi-synthetic medium. J. Gen. Microbiol. 137 : 1155-1162.
- Davey et al. (1996) Flow cytometry and cell sorting of heterogeneous microbial populations: the importance of single-cell analysis. Microbiological Reviews. 60 : 641-696.
- Dudley (1991) Journal of Clinical Immunoassay 14 : 77-82.
- Garcia-Armesto et al. (1993) Modem microbiological methods for foods : colony count and direct count methods. A review. Microbiologia SEM. 9 : 1-13.
- Grant et al. (1998) Isolation of Mycobacterium paratuberculosis from milk by immunomagnetic séparation. Applied and Environmental Microbiology. 64 : 3153-3158.
- Gregori et al. (2001) Resolution of viable and membrane-compromised bacteria in freshwater and marine waters based on analytical flow cytometry and nucleic acid double staining. Applied and Environmental Microbiology. 67 : 4662-4670.
- Haugland (1995) Detecting enzymatic activity in cell using fluorogenic substrates. Biotechnology and Histochemistry. 70: 243-251.
- House et al. (2001) Current Opinion in Infectious Disease. 14 : 573-578.
- Humbert et al. (1989) Rapid identification of Salmonella from poultry meat products by using "Mucap test". Int. J. Food Microbiology. 8 : 79-83.
- lannelli et al. (1998). Simultaneous identification of antibodies to Brucella abortus and Staphylococcus aureus in milk samples by flow cytometry. Journal of Clinical Microbiology. 36 : 802-806.
- Kramer et Jones (1969) Media selective for Listeria monocytogenes. Journal of Applied Bacteriology. 32 : 381-394.
- Lopez-Amoros et al. (1995) Flow cytometric assessment of Escherichia coli and Salmonella starvation-survival in seawater using Rhodamine 123, Propidium iodide and oxonol. Applied and Environmental Microbiology. 61: 2521-2526.
- Manafi M. (2000) New developments in chromogenic and fluorogenic culture média. International Journal of Food Microbiology. 60. 205-218.
- Mason et al. (1995) Journal of Applied Bacteriology. 78 : 309-315.
- McClelland et al. (1994) Detection of Salmonella typhimurium in dairy products with flow cytometry and monoclonal antibodies. Applied and Environmental Microbiology. 60:4255-4262.
- Moter et al. (2000) Fluorescence in situ hybridization (FISH) for direct visualization of microorganisms. Journal of Microbiological Methods. 41: 85-112.
- Nebe-von Caron et al. (1995) Viability assement of bacteria in mixed populations using flow cytometry. J. Microsc. 179 : 55-66.
- Nedergaard et al. (1990) Dicarboxy-dichlorofluorescein : a new fluorescent probe for measuring acidic intracellular pH. Anal. Biochem. 187 : 109-114.
- Notermans et al. (1991) Phosphatidylinositol-specific phospholipase C activity as a marker to distinguish between pathogenic and non-pathogenic Listeria species. Applied and Environmental Microbiology. 57: 2666-2670.
- Olsson et al. (1991). Identification of Salmonellae with the 4-methylumbelliferyl caprilate fluorescence test. J. Clin. Microbiol. Vol 29, p 2631.
- Ouyang et al. (1999) Promoter analysis of the cap8 operon, involved in type 8 capsular polysaccharide production in Staphylococcus aureus. J. Bacteriol. 181 : 1155-1162.
- Perez-Pons et al. (1995) Induction and preliminary characterization of intracellular beta-glucosidases from a cellulolytic Streptomyces strain. FEMS Microbiological Letters. 128 : 235-239.
- Pinder et al. (1994) Rapid immunoassay for pathogenic Salmonella organisms by immunofluorescence flow cytometry. Journal of Microscopy. 176 : 17-22.
- Robinson J.P. (1999) Overview of flow cytometry. In Current Protocols in Cytometry 11.1.1-11.1.4.
- Salzman et al. (1982) Rapid identification of microorganisms by circular-intensity differential scaterring. Appl. Env. Microbiol. 44 : 1081-1085.
- Seo et al. (1998) Immunomagnetic séparation and flow cytometry for rapid detection of Escherichia coli 0157 :H7. J. Food Protect. 61 : 812-816.
- Sheagren J. N. (1984) Staphylococcus aureus. The persistent pathogen. N. Engl. J. Med. 310: 1368-1373 et 1437-1442.
- Skjerve et al. (1991). Immunomagnetic séparation of Salmonella from foods. International Journal of Microbiology. 14 : 11-18.
- Skjerve et al. (1990) Detection of Listeria monocytogenes in foods by immunomagnetic séparation. Applied and Environmental Microbiology. 56 : 3478-3481.
- Sompolinsky et al. (1985) J. Clin Microbiol. 22 : 828-834.
- Soro et al. (1990) J. Clin. Microbiol. 28 : 2707-2710.
- Steen (1986) Simultaneous separate detection of flow angle and large angle light scaterring in an arc lamp-based flow cytometer. Cytometry. 7 : 445-449.
- Swaminathan et al. (1995) in Muray et al. Eds Manuel of Clinical Microbiology. American Society of Microbiology. Washington DC.
- Tomayasu et al. (1998) Improvement of the immunomagnetic séparation method selective for Escherichia coli 0157 strains. Applied and Environmental Microbiology. 64 : 376-382.
- Tortorello et al. (1994) Antibody-direct epifluorescent filter technique for rapid direct enumeration of Escherichia coli 0157 :H7 in beef. Applied and Environmental Microbiology. 60 : 3553-3559.
- Tortorello et al. (1998) Quantitative analysis and isolation of Escherichia coli 0157:H7 in a food matrix using flow cytometry and cell sorting. FEMS Immunology in Medical Microbiology. 19 : 267-274.
- Venturi et al. (2002) Extracellular beta-D-glucosidase from Chaetomium thermophilum var. coprophilum: production, purification and some biochemical properties. Journal of Basic Microbiology. 42 : 55-66.
- Vlieger et al. (1992) Quantitation of polymerase chain reaction products by hybridization-based assays with fluorescent, colorimetric, or chemiluminescent detection. Analytical biochemistry 205 : 1-7.
- Wallner et al. (1995) Flow cytometric analysis of activated sludge with rRNA-targeted probes. Appl. Environ. Microbiol. 61 : 1859-1866.
- Yang et al. (1996) Biol. Pharma Bull. 19 : 701-704.

## Revendications

1. Procédé de détection et de comptage de microorganismes dans un échantillon comprenant les étapes de :
a) enrichissement sélectif du microorganisme recherché dans l'échantillon,
b) induction ou activation d'au moins une activité enzymatique dudit microorganisme,
c) concentration immunomagnétique du microorganisme conditionné,
d) marquage fluorescent du microorganisme concentré obtenu après concentration immunomagnétique et réalisé par l'ajout au milieu contenant ledit microorganisme d'au moins un substrat contenant une partie spécifique de l'activité enzymatique à révéler et une partie marqueur, la transformation du substrat ayant lieu à l'intérieur dudit microorganisme et le produit fluorescent étant retenu dans ledit microorganisme, et
e) détection et analyse de la fluorescence permettant la numération ou le comptage des microorganismes recherchés par une technique choisie dans le groupe comprenant la cytométrie en flux, la cytométrie sur filtre et la microscopie de fluorescence.

2. Procédé selon la revendication 1, **caractérisé en ce que** l'étape d'enrichissement est réalisée dans une composition comprenant :
- du pyruvate de sodium à une concentration comprise entre 1 à 20 g/L, de préférence entre 1 à 10 g/L et plus préférentiellement 4 à 6 g/L,
- du thiosulfate de sodium à une concentration comprise entre 0,5 et 5 g/L, de préférence entre 0,5 et 3 g/L et plus préférentiellement encore environ 2g/L,
- de la catalase à une concentration comprise entre 500 et 20 000 u/L, de préférence entre 2 000 et 8000 u/L et plus préférentiellement encore environ 5000 u/L.

3. Procédé selon la revendication 2, **caractérisé en ce que** ladite composition comprend en outre au moins un antibiotique.

4. Procédé selon l'une des revendications 1 à 3, **caractérisé en ce que** l'étape d'induction d'au moins une activité enzymatique spécifique du microorganisme recherché comprend l'ajout au milieu d'enrichissement du microorganisme d'au moins un substrat non fluorescent spécifique de ladite ou desdites enzyme (s).

5. Procédé selon la revendication 4, **caractérisé en ce que** les étapes a) et b) peuvent être réalisées simultanément.

6. Procédé selon la revendication 4 ou 5, **caractérisé en ce que** l'étape c) peut avoir lieu avant l'étape b) ou l'étape c) peut avoir lieu après l'étape d).

7. Procédé selon l'une des revendications 1 à 3, **caractérisé en ce que** l'étape d'induction, dans le cas où le microorganisme recherché est une bactérie Gram+, comprend de plus une étape d'induction d'au moins un antigène de surface caractéristique du microorganisme recherché comprenant l'ajout au milieu d'enrichissement du microorganisme d'un extrait de levure à une concentration comprise entre 5 et 50 g/L, de préférence entre 10 et 20 g/L et plus préférentiellement encore environ 10 g/L.

8. Procédé selon l'une des revendications 1 à 7, **caractérisé en ce que** l'étape de concentration immunomagnétique comprend les étapes de :
a) mise en contact du microorganisme recherché, présent dans le milieu d'induction, avec un anticorps dirigé contre un antigène spécifique de ce microorganisme, ledit anticorps étant greffé sur une bille magnétique,
b) séparation des complexes billes-anticorps-microorganisme du milieu,
c) séparation du microorganisme du reste du complexe.

9. Procédé selon la revendication 8, **caractérisé en ce que** l'anticorps greffé sur une bille magnétique est dirigé contre un anticorps lui-même dirigé contre un antigène spécifique du microorganisme recherché.

10. Procédé selon la revendication 8 ou 9, **caractérisé en ce que** les billes magnétiques ont un diamètre compris entre 1 et 20 µm, de préférence entre 2 et 8µm.

11. Procédé selon l'une des revendications 1 à 10, **caractérisé en ce que** le marquage fluorescent des microorganismes recherchés est réalisé par l'ajout au milieu contenant lesdits microorganismes d'au moins un substrat comprenant une partie spécifique de l'activité enzymatique à révéler et une partie marqueur.

12. Procédé selon la revendication 11, **caractérisé en ce que** la partie marqueur est constituée d'un marqueur fluorogène excité à 488 nm choisi dans le groupe comprenant les xanthènes, les acridines, les phycobiliprotéines, la cyanine et l'esculine.

13. Procédé selon la revendication 11 ou 12, **caractérisé en ce que** la partie du substrat spécifique de l'activité enzymatique à révéler est choisie parmi un acide gras, d'un ose, d'un phosphate, d'un peptide et/ou d'un sulfate.

14. Procédé selon l'une des revendications 1 à 13, **caractérisé en ce que** la détection et l'analyse de la fluorescence permettant la numération des microorganismes est réalisée par par une technique choisie dans le groupe consistant en la cytométrie en flux, la cytométrie sur filtre et la microscopie de fluorescence.

15. Procédé selon l'une des revendications 1 à 14, **caractérisé en ce que** les étapes a), b), c), d) et e) telles que définies dans la revendication 1 sont précédées d'une étape de filtration de l'échantillon à analyser.

16. Procédé selon la revendication 15, **caractérisé en ce que** la filtration réalisée au moyen d'un filtre dont la porosité est comprise entre 20 et 150 microns, de préférence entre 30 et 100 microns et plus préférentiellement encore d'environ 63 microns.

17. Procédé selon la revendication 15, **caractérisé en ce que** la filtration est réalisée sur une membrane présentant une porosité comprise entre 0,2 et 10µm, de préférence entre 0,2 et 5 µm et plus préférentiellement encore entre 0,2 et 0,5µm.

18. Kit pour la mise en oeuvre du procédé de détection et de comptage d'un microorganisme selon l'une des revendications 1 à 17, comprenant :
- un milieu d'enrichissement sous une forme liquide ou déshydratée comprenant :
- une composition nutritive permettant la multiplication dudit microorganisme, et
- une composition de revivification sélective dudit microorganisme comprenant :
- du pyruvate de sodium à une concentration comprise entre 1 à 20 g/L, de préférence entre 1 à 10 g/L,
- du thiosulfate de sodium à une concentration comprise entre 0,5 et 5 g/L, de préférence entre 0,5 et 3 g/L,
- de la catalase à une concentration comprise entre 500 et 20 000 u/L, de préférence entre 2 000 et 8 000 u/L,
- un sac plastique doublé d'un filtre pleine-page présentant une porosité d'environ 63 µm,
- des billes magnétiques telles que définies dans la revendication 10, un ou plusieurs substrats tels que définis dans la revendication 11 sous forme lyophylisée, des solvants appropriés.

19. Kit selon la revendication 18, **caractérisé en ce que** le milieu d'enrichissement comprend de plus au moins un agent antimicrobien.

## Claims

1. A method for detecting and counting microorganisms in a sample, comprising the steps of:
a) selective enrichment of the sought microorganism in the sample,
b) induction or activation of at least one enzymatic activity of said microorganism,
c) immunomagnetic concentration of the conditioned microorganism,
d) fluorescent marking of the concentrated microorganism after immunomagnetic concentration and achieved by adding to the medium containing said microorganism, at least one substrate containing a specific portion of the enzymatic activity to be revealed and a marker portion, the transformation of the substrate occurring inside said microorganism and the fluorescent product being retained in said microorganism, and
e) detection and analysis of the fluorescence allowing numeration or counting of the sought microorganisms by a technique selected from the group comprising flow cytometry, filter cytometry and fluorescence microscopy.

2. The method according to claim 1, **characterized in that** the enrichment step is carried out in a composition comprising:
- sodium pyruvate at a concentration comprised between 1 and 20 g/L, preferably between 1 and 10 g/L and more preferentially 4 to 6 g/L,
- sodium thiosulfate at a concentration comprised between 0.5 and 5 g/L, preferably between 0.5 and 3 g/L, and still more preferentially about 2 g/L,
- catalase at a concentration comprised between 500 and 20,000 u/L, preferably between 2,000 and 8,000 u/1, and still more preferentially about 5,000 u/L.

3. The method according to claim 2, **characterized in that** said composition further comprises at least one antibiotic.

4. The method according to any of claims 1 to 3, **characterized in that** the step for inducing at least one specific enzymatic activity of the sought microorganism comprises the addition to the enrichment medium for the microorganism, of at least one non-fluorescent substrate specific to said enzyme(s).

5. The method according to claim 4, **characterized in that** the steps a) and b) may be carried out simultaneously.

6. The method according to claim 4 or 5, **characterized in that** step c) may take place before step b) or step c) may take place after step d).

7. The method according to any of claims 1 to 3, **characterized in that** the induction step in the case when the sought microorganism is a Gram+ bacterium, further comprises a step for inducing at least one characteristic surface antigen of the sought microorganism comprising the addition to the enrichment medium of the microorganism of a yeast extract at a concentration comprised between 5 and 50 g/L, preferably between 10 and 20 g/L, and still more preferentially about 10 g/L.

8. The method according to any of claims 1 to 7, **characterized in that** the immunomagnetic concentration step comprises the steps of:
a) putting the sought microorganism present in the induction medium in contact with an antibody directed against the specific antigen of this microorganism, said antibody being grafted on a magnetic bead,
b) separating the beads-antibody-microorganism complexes from the medium,
c) separating the microorganism from the remainder of the complex.

9. The method according to claim 8, **characterized in that** the antibody grafted on a magnetic bead is directed against an antibody itself directed against a specific antigen of the sought microorganism.

10. The method according to claim 8 or 9, **characterized in that** the magnetic beads have a diameter comprised between 1 and 20 µm, preferably between 2 and 8 µm.

11. The method according to any of claims 1 to 10, **characterized in that** the fluorescent marking of the sought microorganisms is achieved by adding to the medium containing said microorganisms, at least one substrate comprising a portion specific to the enzymatic activity to be revealed and a marker portion.

12. The method according to claim 11, **characterized in that** the marker portion consists of a fluorogenic marker excited at 488 nm, selected from the group comprising xanthenes, acridines, phycobiliproteins, cyanine, and esculin.

13. The method according to claim 11 or 12, **characterized in that** the portion of the substrate specific to the enzymatic activity to be revealed is selected from a fatty acid, an ose sugar, a phosphate, a peptide and/or a sulphate.

14. The method according to any of claims 1 to 13, **characterized in that** detection and analysis of the fluorescence allowing numeration of the microorganisms, is carried out by a technique selected from the group consisting in flow cytometry, filter cytometry and fluorescence microscopy.

15. The method according to any of claims 1 to 14, **characterized in that** steps a), b), c), d) and e) as defined in claim 1, are preceded by a step for filtering the sample to be analyzed.

16. The method according to claim 15, **characterized in that** filtration is carried out by means of a filter, the porosity of which is comprised between 20 and 150 microns, preferably between 30 and 100 microns, and still more preferentially about 63 microns.

17. The method according to claim 15, **characterized in that** filtration is carried out on a membrane having a porosity comprised between 0.2 and 10 µm, preferably between 0.2 and 5 µm, and still more preferentially between 0.2 and 0.5 µm.

18. A kit for applying the microorganism detecting and counting method according to any of claims 1 to 17, comprising:
- an enrichment medium in a liquid or dehydrated form comprising:
- a nutritive composition allowing multiplication of said organism, and
- a composition for selectively reviving said microorganism, comprising:
- sodium pyruvate at a concentration comprised between 1 to 20 g/L, preferably between 1 to 10 g/L,
- sodium thiosulphate at a concentration comprised between 0.5 and 5 g/L, preferably between 0.5 and 3 g/L,
- catalase at a concentration comprised between 500 and 20,000 u/L, preferably between 2,000 and 8,000 u/L,
- a plastic bag lined with a full page filter having a porosity of about 63 µm,
- magnetic beads as defined in claim 10, one or more substrates as defined in claim 11 in a freeze-dried form, suitable solvents.

19. The kit according to claim 18, **characterized in that** the enrichment medium further comprises at least one antimicrobial agent.

## Patentansprüche

1. Verfahren zur Detektion und Zählung von Mikroorganismen in einer Probe, die Schritte umfassend:
a) selektive Anreicherung des gesuchten Mikroorganismus in der Probe,
b) Induzierung oder Aktivierung mindestens einer enzymatischen Aktivität des Mikroorganismus,
c) immunomagnetische Konzentration des konditionierten Mikroorganismus,
d) Flüoreszenzmarkierung des nach immunomagnetischer Konzentration hergestellten konzentrierten Mikroorganismus und durchgeführt durch Hinzufügen von mindestens einem Substrat, das einen spezifischen Teil der hervorzurufenden enzymatischen Aktivität und einen Markerteil enthält, zum den Mikroorganismus enthaltenden Milieu, wobei die Transformation des Substrats innerhalb des Mikroorganismus stattfindet und das fluoreszierende Produkt im Mikroorganismus zurückgehalten wird, und
e) Detektion und Analyse der Fluoreszenz, die die zahlenmäßige Ermittlung oder Zählung der gesuchten Mikroorganismen durch eine Technik erlauben, die ausgewählt ist aus der Gruppe, die die Durchflusszytometrie, die Zytometrie über Filter und die Fluoreszenzmikroskopie umfasst.

2. Verfahren nach Anspruch 1, **dadurch gekennzeichnet, dass** der Schritt der Anreicherung in einer Zusammensetzung durchgeführt wird, die umfasst:
- Natriumpyruvat in einer Konzentration zwischen 1 bis 20 g/L inklusive, vorzugsweise zwischen 1 bis 10 g/L und besonders vorzugsweise 4 bis 6 g/L,
- Natriumthiosulfat in einer Konzentration zwischen 0,5 und 5 g/L inklusive, vorzugsweise zwischen 0,5 und 3 g/L und besonders vorzugsweise auch zirka 2 g/L,
- Katalase in einer Konzentration zwischen 500 und 20.000 u/L inklusive, vorzugsweise zwischen 2.000 und 8.000 u/L und besonders vorzugsweise auch zirka 5.000 u/L.

3. Verfahren nach Anspruch 2, **dadurch gekennzeichnet, dass** die Zusammensetzung weiterhin mindestens ein Antibiotikum umfasst.

4. Verfahren nach einem der Ansprüche 1 bis 3, **dadurch gekennzeichnet, dass** der Schritt der Induzierung mindestens einer spezifischen enzymatischen Aktivität des gesuchten Mikroorganismus das Hinzufügen zum Anreicherungsmilieu des Mikroorganismus mindestens eines nicht fluoreszierenden Substrats umfasst, das für das oder die Enzym/e spezifisch ist.

5. Verfahren nach Anspruch 4, **dadurch gekennzeichnet, dass** die Schritte a) und b) gleichzeitig durchführbar sind.

6. Verfahren nach Anspruch 4 oder 5, **dadurch gekennzeichnet, dass** der Schritt c) vor dem Schritt b) stattfinden kann oder der Schritt c) nach dem Schritt d) stattfinden kann.

7. Verfahren nach einem der Ansprüche 1 bis 3, **dadurch gekennzeichnet, dass** der Schritt der Induzierung in dem Fall, wenn der gesuchte Mikroorganismus eine gram-positive Bakterie ist, zusätzlich einen Schritt der Induzierung mindestens eines für den gesuchten Mikroorganismus charakteristischen Oberflächenantigens zum Anreicherungsmilieu des Mikroorganismus umfasst, der das Hinzufügen eines Hefeextrakts in einer Konzentration zwischen 5 und 50 g/L inklusive, vorzugsweise zwischen 10 und 20 g/L und besonders vorzugsweise auch zirka 10 g/L umfasst.

8. Verfahren nach einem der Ansprüche 1 bis 7, **dadurch gekennzeichnet, dass** der Schritt der immunomagnetischen Konzentration die Schritte umfasst:
a) Herstellung eines Kontakts des im Induzierungsmilieu vorhandenen gesuchten Mikroorganismus mit einem Antikörper, der gegen einen spezifischen Antigen dieses Mikroorganismus gerichtet ist, wobei der Antikörper auf eine Magnetkugel aufgepfropft ist,
b) Separierung der Komplexe Kugeln-Antikörper-Mikroorganismus vom Milieu,
c) Separierung des Mikroorganismus vom restlichen Komplex.

9. Verfahren nach Anspruch 8, **dadurch gekennzeichnet, dass** der auf eine Magnetkugel gepfropfte Antikörper gegen einen Antikörper gerichtet ist, der selbst gegen einen spezifischen Antigen des gesuchten Mikroorganismus gerichtet ist.

10. Verfahren nach Anspruch 8 oder 9, **dadurch gekennzeichnet, dass** die Magnetkugeln einen Durchmesser zwischen 1 und 20 µm inklusive, vorzugsweise zwischen 2 und 8 µm haben.

11. Verfahren nach einem der Ansprüche 1 bis 10, **dadurch gekennzeichnet, dass** die Fluoreszenzmarkierung der gesuchten Mikroorganismen durch Hinzufügen mindestens eines Substrats, das einen spezifischen Teil der hervorzurufenden enzymatischen Aktivität und einen Markerteil umfasst, zu dem die Mikroorganismen enthaltenden Milieu durchgeführt wird.

12. Verfahren nach Anspruch 11, **dadurch gekennzeichnet, dass** der Markerteil von einem bei 488 nm erregten fluorogenen Marker gebildet wird, der aus der Gruppe ausgewählt ist, die die Xanthene, die Acridine, die Phycobiliproteine, das Cyanin und das Esculin umfasst.

13. Verfahren nach Anspruch 11 oder 12, **dadurch gekennzeichnet, dass** der Teil des für die hervorzurufende enzymatische Aktivität spezifischen Substrats aus einer Fettsäure, einer Ose, einem Phosphat, einem Peptid und/oder einem Sulfat ausgewählt ist.

14. Verfahren nach einem der Ansprüche 1 bis 13, **dadurch gekennzeichnet, dass** die Detektion und die Analyse der Fluoreszenz, die eine zahlenmäßige Ermittlung der Mikroorganismen erlaubt, anhand einer Technik durchgeführt wird, die aus der Gruppe ausgewählt ist, die aus der Durchflusszytometrie, der Zytometrie über Filter und der Fluoreszenzmikroskopie besteht.

15. Verfahren nach einem der Ansprüche 1 bis 14, **dadurch gekennzeichnet, dass** den Schritten a), b), c), d) und e), so wie in Anspruch 1 definiert, ein Filtrationsschritt der zu analysierenden Probe vorangeht.

16. Verfahren nach Anspruch 15, **dadurch gekennzeichnet, dass** die Filtration mit einem Filter durchgeführt wird, dessen Porosität zwischen 20 und 150 Mikron inklusive ist, vorzugsweise zwischen 30 und 100 Mikron und besonders vorzugsweise auch zirka 63 Mikron.

17. Verfahren nach Anspruch 15, **dadurch gekennzeichnet, dass** die Filtration über einer Membran durchgeführt wird, die eine Porosität zwischen 0,2 und 10 µm inklusive aufweist, vorzugsweise zwischen 0,2 und 5 µm und besonders vorzugsweise auch zwischen 0,2 und 0,5 µm.

18. Kit zur Durchführung des Verfahrens zur Detektion und Zählung eines Mikroorganismus nach einem der Ansprüche 1 bis 17, umfassend:
- ein Anreicherungsmilieu in flüssiger oder dehydrierter Form, umfassend:
- eine nährende Zusammensetzung, die die Vermehrung des Mikroorganismus erlaubt, und
- eine Zusammensetzung zur selektiven Regenerierung des Mikroorganismus, umfassend:
- Natriumpyruvat in einer Konzentration zwischen 1 bis 20 g/L inklusive, vorzugsweise zwischen 1 bis 10 g/L,
- Natriumthiosulfat in einer Konzentration zwischen 0,5 und 5 g/L inklusive, vorzugsweise zwischen 0,5 und 3 g/L,
- Katalase in einer Konzentration zwischen 500 und 20.000 u/L inklusive, vorzugsweise zwischen 2.000 und 8.000 u/L,
- einen mit einem Ganzseitenfilter gefütterten Kunststoffbeutel mit einer Porosität von zirka 63 µm,
- Magnetkugeln, wie in Anspruch 10 definiert, ein oder mehrere Substrate, wie in Anspruch 11 definiert, in lyophylisierter Form, geeignete Lösungsmittel.

19. Kit nach Anspruch 18, **dadurch gekennzeichnet, dass** das Anreicherungsmilieu zusätzlich mindestens ein antimikrobielles Mittel umfasst.
